(19) Europäisches Patentamt
European Patent Office
Office européen des brevets

(11) Publication number: 0 308 499 B1

# (12) EUROPEAN PATENT SPECIFICATION

(45) Date of publication of patent specification: 07.01.93

(51) Int. Cl.5: C12N 15/52, C12N 9/00, C12N 9/54, C12N 9/96, C12N 15/57

(21) Application number: 88903692.7

(22) Date of filing: 30.03.88

(86) International application number: PCT/US88/01078

(87) International publication number: WO 88/07578 (06.10.88 88/22)

## (54) SUBSTRATE ASSISTED CATALYSIS.

(30) Priority: 02.04.87 US 34085

(43) Date of publication of application: 29.03.89 Bulletin 89/13

(45) Publication of the grant of the patent: 07.01.93 Bulletin 93/01

(84) Designated Contracting States: AT BE CH DE FR GB IT LI LU NL SE

(56) References cited:

Science, vol. 237, 24 July 1987, (Washington, DC, US), P. Carter et al.: "Engineering enzyme specificity by "Substrate-Assited Catalysis" ", pp. 394-399

Proc. Natl. Acad. Sci. USA, vol. 84, March 1987, (Washington, DC, US), J.A. Wells et al.: "Designing substrate specificity by protein engineering of electrostatic interactions", pp. 1219-1223

(73) Proprietor: GENENTECH, INC.
480 Point San Bruno Boulevard
San Francisco, CA 94080(US)

(72) Inventor: WELLS, James, Allen
65 Otay Avenue
San Mateo, CA 94403(US)
Inventor: CARTER, Paul, John
2074 18th Avenue
San Francisco, CA 94116(US)

(74) Representative: Armitage, Ian Michael et al
MEWBURN ELLIS & CO. 2/3 Cursitor Street
London EC4A 1BO(GB)

EP 0 308 499 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

The present invention relates to novel enzyme mutants which are derived from a precursor enzyme by replacing or modifying at least one catalytic functional group of an amino acid residue in a precursor enzyme. Such mutant enzymes have a catalytic preference for substrates which provide the replaced or modified functional group or its equivalent such that the substrate, in essence, together with the enzyme mutant, assists in its own catalysis.

### PREFILING DISCLOSURES

Enzymes are polypeptides which catalyze a wide variety of chemical reactions. It is generally accepted that enzymatic catalysis requires that the substrate bind to the enzyme in the region of the enzyme's active site such that the specific region being acted upon by the enzyme is distorted into a configuration approximating the transition state of the reaction being catalyzed. In many cases the specific site of catalysis within the substrate must be oriented so that specific residues of the enzyme involved in catalysis can act on the bound and distorted substrate. Thus, within the active site, amino acid residues can generally be characterized as those primarily involved in substrate binding and hence determinative of substrate specificity and those involved primarily with the actual chemical catalysis, e.g., those involved in proton or electron transfer or nucleophilic or electrophilic attack on the substrate.

A wide variety of classical methods have been used to deduce the binding and catalytic residues in the active site of an enzyme. For example, the x-ray crystal structures of the serine endoprotease subtilisin containing covalently bound peptide inhibitors (Robertus, J.D., et al. (1972) Biochemistry 11, 2439-2449), product complexes (Robertus, J.D., et al. (1972) Biochemistry 11, 4293-4303), and transition state analogs (Matthews, D.A., et al. (1975) J. Biol. Chem. 250, 7120-7126; Poulos, T.L. et al. (1976) J. Biol. Chem. 251, 1097-1103), which have been reported have provided information regarding the active site of subtilisin including the amino acid residues involved in substrate binding and catalytic activity. In addition, a large number of kinetic and chemical modification studies have been reported for subtilisin which have also aided in deducing the substrate binding and catalytic residues of subtilisin (Philip, M., et al. (1983) Mol. Cell. Biochem. 51, 5-32; Svendsen, I.B. (1976) Carlsberg Res. Comm. 41, 237-291; Markland, F.S. et al. (1971) In The Enzymes Ed. Boyer, P.D., Academic Press, New York, Vol. 3, pp. 561-608). In most cases where the chemical modification was to a catalytic amino acid residue, the enzymatic activity of the enzymes modified was destroyed or severely impaired (Fersht, A. (1977) "Enzyme Mechanism and Structure", William Freeman, San Francisco, California, pp. 201-205). In two reported examples, chemical modification of the active site serine of subtilisin resulted in the replacement of the serine-OH with -SH which produced a modified enzymatic activity (Neet, K.E., et al. (1968) J. Bio. Chem. 248, 6392-6401; Polgar, L., et al. (1967) Biochemistry 6, 610-620). Most chemical modifications of catalytic residues, however, necessarily maintain or increase the effective side chain volume of the amino acid modified and consequently maintain or decrease the effective volume within which the catalytic residues must function.

The recent development of various in vitro techniques to manipulate the DNA sequences encoding naturally-occurring polypeptides as well as recent developments in the chemical synthesis of relatively short sequences of single and double stranded DNA has resulted in the reported synthesis of various enzymes wherein specific amino acid residues have been substituted with different amino acids (Ulmer, K.M. (1983) Science 219, 666-671).

There are several reported examples where a catalytic residue of a particular enzyme has been substituted with a different amino acid. Some of these references describe the replacement of a catalytic amino acid with an amino acid having a side chain functional group different from that of the catalytic amino acid being replaced e.g. substitution of a neutral polar side chain moiety for a side chain moiety containing an acid group or substitution of one nucleophilic side chain moiety with a different nucleophilic moiety. Others describe replacements where the side chain functional group of a catalytic residue remained constant but the position of that functional group was moved within the active site.

For example, Aspartate-102 of eucaryotic trypsinogen is reported to be a catalytic residue required for endoprotease activity. Roczniak, S.O., et al. (1985), J. Cell Biochem 9B (Abstracts) p. 87 briefly report the substitution of asparagine for aspartate at position 102. In this case, the carboxylate of aspartic acid was effectively substituted with the polar neutral side chain of asparagine which reportedly resulted in a dramatic decrease in kcat.

Dalbadie-McFarland, G., et al. (1982) PNAS (USA) 79, 6439-6413, report the inversion of the ser-thr

diad of the β-lactamase gene contained in plasmid pBR322. This inversion resulted in the conversion of the catalytically active Serine-70 to Threonine and reportedly produced a mutant with an ampicillin-sensitive phenotype.

The substitution of Serine-70 in β-lacatamase with cysteine is reported by Sigal, I.S., et al. (1984) J. Bio. Chem. 259, 5327-5332. This replacement of an active site serine by a cysteine residue results in the net substitution of an -OH group by an -SH group, each of which can be effective nucleophiles. The thiol-containing β-lactamase reportedly catalyses the hydrolysis of β-lactams with a substrate specificity that is distinct from that of the wild type enzyme. For benzyl penicillin and ampicillin, the Km values are similar to wild type values although the kcat values are 1-2% that of a wild type enzyme. However, when reacted with the cephalosporin nitrocefin, the Km is greater than 10 fold that of the wild type and the kcat is at least as large as the kcat for the wild type enzyme.

In Strauss, et al. (1985) PNAS (USA) 82, 2272-2276, triosphosphate isomerase was reportedly modified to replace glutamic acid at position 165 with aspartic acid. This replacement does not alter the chemical nature of the side chain at position 165 but rather moves the catalytic carboxyl group at that position, in essence, by the removal of a methylene group from glutamic acid. The kcat for different substrates was dramatically altered by this mutation leading the author to conclude that glutamic acid at position 165 is critical for proton shuttling during catalysis and further suggesting that this residue makes only a small contribution to the binding of the reaction intermediates.

The substitution of Serine-102 in the active site of alkaline phosphatase with cysteine is reported by Ghosh, S.S., et al. (1986) Science 231, 154-148. The resulting thiol enzyme catalyzes the hydrolysis of a variety of phosphate monoesters. The authors hypothesize, however, based on the observed catalytic effeciency of the thiol containing enzyme, that the serine to cysteine mutation results in a change in the rate-determining step of catalysis from dephosphorylation to the formation of a phosphoryl-enzyme intermediate.

The substitution of different amino acids for putative catalytic residues in various enzymes has been directed to the determination of whether these residues are primarily involved in catalysis rather than substrate binding. In several reported cases, however, the expected result was not obtained. In Gardell, S.J. et al. (1985) Nature 317, 551-555, Tyrosine-248 in carboxypeptidase A from rat was substituted with phenylalanine. Tyrosine-248 had previously been thought to play a role in catalysis through its phenolic side chain. The particular substitution described removed the putative phenolic hydroxide moiety and substituted a phenyl moiety. The authors report that the catalytic reactivity of the wild type enzyme compared to the substituted enzyme containing phenylalanine at position 248, for certain substrates, indicated that Tyrosine-248 was not obligatory for the hydrolysis of peptide substrates. Rather, the authors suggests that the Tyrosine-248 hydroxyl group participates in substrate binding rather than catalysis.

Similarly, Threonine-113 in dihydrofolate reductase from E.coli is a strictly conserved residue at the dihydrofolate binding site which interacts with a second conserved residue, Aspartate-27, via a hydrogen bond and presumably with the substrate dihydrofolate indirectly through a water molecule (Jin-Tann Chen, et al. (1985) J. Cell. Biochem 29, 73-82). Since Aspartate-27 is also conserved and involved in catalysis, this suggested to the authors that Threonine-113 could be required for proton transfer during catalysis. The authors report the substitution of Threonine-113 with valine and conclude that Threonine-113 is not involved in catalysis since there is no loss of catalytic efficiency upon substitution with valine.

Schultz, S.C., et al. (1986) PNAS (USA) 83, 1588-1592, report the substitution of threonine-71 in class A β-lactamase with all possible amino acid substitutions to determine the role of this residue. Threonine-71 is a residue in the conserved triad Ser-Thr-Xaa-Lys. The results obtained by these authors suggests that Threonine-71 is not essential for binding or catalysis, as expected, but is important for stability of the β-lactamase protein.

Much of the work involving the substitution of different amino acids in various enzymes has been directed to the substitution of amino acid residues involved in substrate binding. Examples include the substitution of single amino acids within the active site of tyrosyl-tRNA synthetase (Cysteine-35→Serine, Winter, G. et al. (1982) Nature 299, 756-758; Cysteine-35→Glycine, Wilkinson, A.J. et al. (1983) Biochemistry 22, 3581-3586; and Threonine-51→Alanine and Threonine-51→Proline, Wilkinson A.J. et al. (1984) Nature 307, 187-183).

Other examples of substitutions of amino acids involved in substrate binding include a double mutant of tyrosyl-tRNA synthetase involving Cysteine-35→ Glycine together with Threonine-51→Proline (Carter, P.J. et al. (1984) Cell 38, 835-840); the substitution of glycine residues at positions 216 and 226 of rat pancreatic trypsin with alanine residues to produce two single substitutions and one double substitution (Craik, C.S. et al. (1985) Science 228, 291-297); and the substitution of various non-catalytic residues in dihydrofolate reductase (Villafranca, J.E., et al. (1983) Science 222, 782-788).

Paluh, J.L., et al. (1984) J. Biol. Chemistry 260, 1188-1894, report the substitution of Cysteine-84 with glycine in Serratia marcescens anthranilate synthase Component II. They report that this replacement abolished the glutamine-dependent anthranilate synthase activity but not the ammonium-dependent activity of the enzyme. They also conclude that the mutation provides further evidence for the role of the active site Cysteine-84 in the glutamine amide transfer function of the enzyme. The authors also note, however, that the specific amino acid replacement might cause a relatively minor structural alteration that could abolish a glutamine binding or amide transfer independent of the function of Cysteine-84. It is not clear from this reference whether Cysteine-84 is a residue involved in binding, or actual catalysis.

The substitution of amino acid residue believed to be involved in transition state stabilization of various enzymes have also been reported. Such work has recently been summarized in Fersht, A.R., et al. (1986), Trends in Biochemical Sciences, 11, 321-325.

A reference in another field is Rossman, M.G., et al. (1985) Nature 317, 145-153 wherein the RNA of a human rhino virus is postulated to act as a proton acceptor for the autocatalytic cleavage of the viral coat protein VPO into VP2 and VP4.

The references discussed above are provided solely for their disclosure prior to the filing date of the present case, and nothing herein is to be construed as an admission that the inventors are not entitled to antedate such disclosure by virtue of prior invention or priority based on earlier filed applications.

Based on the above references, however, it is apparent that those skilled in the art have focused on altering enzyme specificity by changing binding residues. It has heretofore not been recognized that residues containing side chains directly involved in catalysis can be substituted with residues containing smaller and catalytically inactive side chains to produce enzyme mutants which are catalytically active with substrates which provide the catalytic function of the replaced residue side chain. Thus, these enzyme mutants have a substrate specificity which is distinguished primarily at the level of catalysis rather than substrate binding.

Accordingly, it is an object herein to provide enzyme mutants wherein at least one catalytic group of an amino acid residue of a precursor enzyme is replaced or modified such that the thus formed mutant enzyme has a preferred catalytic activity for a substrate which is capable of providing the replaced or modified catalytic function when in contact with the mutant enzyme.

It is a further object to provide DNA sequences encoding such enzyme mutants as well as expression vectors containing such mutant DNA sequences.

Still further, another object of the present invention is to provide host cells transformed with such vectors as well as host cells which are capable of expressing such enzyme mutants either intracellularly or extracellularly.

A further object of the present invention is to provide a catalytically active mutant enzyme substrate complex wherein at least one of the catalytic functional group, of the complex is provided by the substrate.

Still further, an object of the present invention is to provide processes wherein the enzyme mutants of the invention are contacted used with modified substrates to bring about desired enzymatic catalysis.

## SUMMARY OF THE INVENTION

The invention includes enzyme mutants not found in nature which are derived from a precursor enzyme by the replacement or modification of at least one catalytic group of an amino acid residue which when in contact with a selected region of a polypeptide substrate functions catalytically therewith. The enzyme mutant so formed is relatively inactive catalytically with the corresponding substrate as compared to the mutant's catalytical activity with a modified substrate formed by replacing or modifying a moiety in a selected region of the precursor enzyme's substrate. This selected region of the substrate is modified to include the catalytic group, or its equivalent which is replaced or modified in the precursor enzyme, such that the enzyme mutant is catalytically active with the modified substrate.

The invention also includes mutant DNA sequences encoding such mutant enzymes, expression vectors containing such mutant DNA sequences and host cells transformed with such vectors which are capable of expressing said enzyme mutants.

The invention also includes a catalytically active enzyme-substrate complex comprising an enzyme mutant and a modified substrate. The enzyme mutant is not found in nature and is derived from a precursor enzyme by the replacement or modification of at least one catalytic group of an amino acid residue which, when in contact with a selected region of a substrate for the precursor enzyme, functions catalytically with such substrate. The enzyme mutant so formed is relatively inactive with the substrate for the precursor enzyme as compared to the enzyme mutant's catalytic activity with a modified substrate. The modified substrate is formed by replacing or modifying a moiety in the selected region of the precursor enzyme's

substrate. This selected region of the substrate is modified to include the catalytic group, or its equivalent, which is replaced or modified in the precursor enzyme such that the enzyme mutant is catalytically active with the modified substrate.

The invention further includes a process comprising contacting an enzyme mutant and a modified substrate to produce substrate assisted catalysis of the modified substrate. In this aspect of the invention, the enzyme mutant is the same as that defined for the enzyme mutant-substrate complex of the invention.

## BRIEF DESCRIPTION OF THE DRAWINGS

Fig. 1 is the DNA and amino acid sequence for B. amylolquefaciens subtilisin.

Fig. 2 depicts catalytic residues of B. amylolquefaciens subtilisin.

Figs. 3A and 3B depict the amino acid sequence of subtilisin as obtained from various sources.

Fig. 3C depicts the conserved residues of B. amylolquefaciens subtilisin when compared to other subtilisin sequences.

Fig. 4 is a schematic diagram showing the substrate binding cleft to subtilisin together with a substrate.

Fig. 5 is a stero view of B. amylolquefaciens subtilisin containing a modeled bound peptide substrate having the sequence L-Phe-L-Ala-L-His-L-Tyr-L-Gly-L-Phe representing residues P4 to P2' of the substrate.

Fig. 6 depicts the plasmid PS4.

Fig. 7A, 7B and 7C depict the pH dependence of hydrolysis of p-nitroanilide peptide substrates by Cys-24/Ala-64 subtilisin.

Fig. 8 depicts the hydrolysis of a polypeptide substrate by Cys-24/Ala-64 subtilisin.

Fig. 9 depicts a stero view of a complex between bovine trypsin and pancreatic trypsin inhibitor complex.

## DETAILED DESCRIPTION OF THE INVENTION

The inventors have discovered that various catalytic groups in amino acid side chains in an enzyme can be replaced or modified to produce a mutant enzyme which is reactive with substrates which contain the replaced or modified catalytic group. The replaced or modified catalytic group is located in the substrate such that it is able to assist, with the mutant enzyme, in the catalysis of the modified substrate.

Specifically, B. amyloliquefaciens subtilisin, an alkaline bacterial protease, has been mutated by modifying the DNA encoding subtilisin to encode the substitution of the catalytic residue His-64 with alanine. As expected, kcat and the catalytic efficiency, as measured by kcat/Km, of this mutant enzyme was significantly reduced as compared to the wild type subtilisin when contacted with substrates readily cleaved by wild type subtilisin. Surprisingly, various substrates containing histidine in the P2 position were preferred by the Ala-64 mutant subtilisin.

Previous studies have focused on altering enzyme specificity by changing residues that bind the substrate to the enzyme. The alternative approach described herein, termed "substrate-assisted catalysis", is applicable to a wide range of enzymes and substrates other than those specifically disclosed herein. In general, the invention is applicable to any enzyme in which part of the enzyme is removed and appropriately supplied by a similar functionality from a bound substrate. In this way substrates are distinguished primarily at the level of catalysis instead of binding, permitting the design of extremely specific enzyme mutants.

As used herein, "enzymes" are polypeptides which either alone or in conjunction with various co-factors catalyze a covalent change in a substrate. Enzymes can be categorized according to a systematic nomenclature and classification which has been adopted on the recommendation of the International Enzyme Commission. Thus, enzymes can be categorized as oxidoreductases (enzymes involved in oxidation reduction reactions), transferases (enzymes involved in the transfer of functional groups), hydrolases (enzymes involved in hydrolytic reactions), lyases (enzymes catalyzing addition reactions to double bonds), isomerases (enzymes involved in isomerization reactions) and ligases (enzymes involved in the formation of bonds with ATP cleavage). See, generally, Lehninger, A.L., Biochemistry, Worth Publishers, Inc., New York, New York (1970), pp. 147-187.

A "precursor enzyme" refers to an enzyme in which a catalytic amino acid residue can be replaced or modified to produce a mutant enzyme. Typically, the DNA sequence encoding the precursor enzyme may be modified to produce a mutant DNA sequence which encodes the substitution of one or more catalytic amino acids in the precursor enzyme amino acid sequence. Suitable modification methods are disclosed herein and in EPO Publication No. 0130756 published January 9, 1985. The precursor enzyme, however, can also be modified by means other than recombinant DNA technology to produce the mutant enzyme of

the invention.

A precursor enzyme may also be a recombinant enzyme which refers to an enzyme for which its DNA has been cloned or to an enzyme in which the cloned DNA sequence encoding an enzyme is modified to produce a recombinant DNA sequence which encodes the substitution, deletion or insertion of one or more amino acids in the sequence of a naturally occurring enzyme. Suitable methods to produce such modifications include those disclosed herein and in EPO Publication No. 0130756. For example, the subtilisin multiple mutant herein containing the substitution of serine at amino acid residue 24 with cysteine and the substitution of histidine at amino and residue 64 with alanine can be considered to be derived from the recombinant subtilisin containing the substitution of cysteine for serine at residue 24. The mutant thus is produced by the substitution of alanine for histidine at residue 64 in the Cys-24 recombinant subtilisin.

Carbonyl hydrolases are enzymes which hydrolyze compounds containing

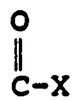

bonds in which X is oxygen or nitrogen. They include naturally-occurring carbonyl hydrolases and recombinant or chemically synthesized carbonyl hydrolases. Naturally occurring carbonyl hydrolases principally include hydrolases, e.g. lipases and peptide hydrolases, e.g. subtilisins or metalloproteases. Peptide hydrolases include α-aminoacylpeptide hydrolase, peptidylamino-acid hydrolase, acylamino hydrolase, serine carboxypeptidase, metallocarboxypeptidase, thiol proteinase, carboxylproteinase and metalloproteinase. Serine, metallo, thiol and acid proteases are included, as well as endo and exo-proteases.

Subtilisins are bacterial carbonyl hydrolases which generally act to cleave peptide bonds of protains or peptides. As used herein, "subtilisin" means a naturally occurring subtilisin or a recombinant subtilisin. A series of naturally occurring subtilisins is known to be produced and often secreted by various bacterial species. Amino acid sequences of the members of this series are not entirely homologous. However, the subtilisins in this series exhibit the same or similar type of proteolytic activity. This class of serine proteases shares a common amino acid sequence defining a catalytic triad which distinguishes them from the chymotrypsin related class of serine proteases. The subtilisins and chymotrypsin related serine proteases both have a catalytic triad comprising aspartate, histidine and serine. In the subtilisin related proteases the relative order of these amino acids, reading from the amino to carboxy terminus is aspartate-histidineserine. In the chymotrypsin related proteases the relative order, however, is histidine-aspartateserine. Thus, subtilisin herein refers to a serine protease having the catalytic triad of subtilisin related proteases.

Carbonyl hydrolases and their genes may be obtained from many procaryotic and eucaryotic organisms. Suitable examples of procaryotic organisms include gram negative organisms such as E. coli or pseudomonas and gram positive bacteria such as micrococcus or bacillus. Examples of eucaryotic organisms from which carbonyl hydrolase and their genes may be obtained include yeast such as S. cerevisiae, fungi such as Aspergillus sp., and mammalian sources such as, for example, Bovine sp. from which the gene encoding the carbonyl hydrolase chymosin can be obtained. As with subtilisins, a series of carbonyl hydrolases can be obtained from various related species which have amino acid sequences which are not entirely homologous between the members of that series but which nevertheless exhibit the same or similar type of biological activity. Thus, carbonyl hydrolase as used herein has a functional definition which refers to carbonyl hydrolases which are associated, directly or indirectly, with procaryotic and eucaryotic sources.

An "enzyme mutant" has an amino acid sequence which is derived from the amino acid sequence of a "precursor enzyme" and has a catalytic preference for a modified substrate as defined herein. The amino acid sequence of the enzyme mutant may be "derived" from the precursor amino acid sequence by the substitution of one or more catalytic amino acid residues of the precursor amino acid sequence. Suitable methods for such manipulation of the precursor DNA sequence include methods disclosed herein and in EPO Publication No. 0130756. Other methods, for example, to directly modify the amino acid side chain of the precursor enzyme may be used provided they produce the catalytic preference for a modified substrate. A "catalytic amino acid residue" is one which contains a catalytic group.

As used herein in connection with enzyme mutants, a "catalytic group" in an enzyme is a functional side chain of an amino acid residue which undergoes a change in charge or chemical bonding state during a reaction sequence and which becomes regenerated at the end of the reaction sequence, or which interacts directly with such a functional side chain to facilitate its change in charge or chemical bonding

state. Catalytic groups typically participate in catalysis by interacting directly or indirectly as a nucleophile, electrophile, acid, base or electron transfer agent with the reactive site of a substrate. Typical catalytic amino acid residues and their respective catalytic groups (shown in parentheses) include: Ser(-OH), Thr(-OH), Cys(-OH), Tyr(-OH), Lys($-NH_2$), Asp($-CO_2H$), Glu($-CO_2H$), His(imidazolyl) and Met($-SCH_3$). See Table I. Thus, for example, catalytic groups for B. amyloliquefaciens subtilisin and as shown in Fig. 2 corresponding to the amino acid position numbers referred to in Fig. 1 comprise the side chains to the amino acids Asp-32, His-64 and Ser-221.

TABLE I

| Precursor Enzyme | | | Modified Substrate | |
|---|---|---|---|---|
| Catalytic Catalytic Residue | Preferred Amino Acid Residue Substitution | Alternate Amino Acid Residue Substitution | Catalytic Group | Equivalent Catalytic Group |
| His | Gly, Ala | Ser | Imidazoyl | $-NH_2$ |
| Lys | Gly, Ala, Ser | Thr, Leu, Asn | $-NH_2$ | Imidazolium |
| Ser | Gly | Ala | -OH | -SH |
| Thr | Gly | Ala | -OH | -SH |
| Cys | Gly | Ala | -SH | -HO |
| Asp | Gly, Ala | Ser | $-CO_2H$ | Imadazoyl, Phenol |
| Glu | Gly, Ala Ser | Thr, Cys | $-CO_2H$ | Imadazoyl, Phenol |
| Tyr | Gly, Ala Ser, Asn, Gln | Thr, Cys | Phenol | -OH, -SH Imidazolium |
| Met | Gly, Ala | Ser, Thr | $-S-CH_3$ | -SH |
| Phe | Gly, Ala, Ser | Leu, Val | Phenyl | $-S-CH_3$, Phenol |
| Trp | Gly, Ala, Ser | Leu, Val | Indole | $-S-CH_3$, Phenol, Phenyl |

As used herein in connection with the enzyme-substrate complexes or processes of the invention, a "catalytic group" in addition to the above definition, includes functional side chains of amino acid residues which aid in stabilizing the transition state of a reaction by interacting directly or indirectly with a polarized or charged transition state. Such transition state stabilization is typically achieved by the formation of salt bridges or the creation of a dipole-dipole interaction (e.g. hydrogen bond formation) between the transition state and the catalytic residues stabilizing it. Typical catalytic amino acid residues involved in transistion state stabilization and their respective catalytic groups (shown in parenthesis) include those catalytic residues of Table I:

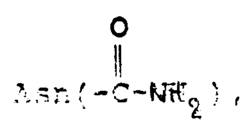

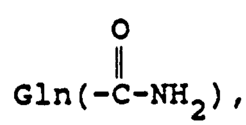

and

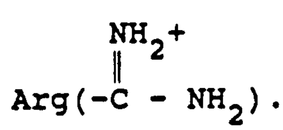

(See Table II). For the B. amyloliquefaciens subtilisin shown in Figs. 1 and 2, a catalytic residue involved in transition state stabilization is Asn-155 which provides a hydrogen bond to stabilize the oxyanion of the tetrahydral intermediate shown in Fig. 2.

## TABLE II

| Precursor Enzyme | | | Modified Substrate | |
|---|---|---|---|---|
| Catalytic Catalytic Residue | Preferred Amino Acid Residue Substitution | Alternate Amino Acid Residue Substitution | Catalytic Group | Equivalent Catalytic Group |
| Asn | Gly, Ala, | Thr, Ser, Cys | O<br>‖<br>$-C-NH_2$ | -OH<br>Imidazoyl |
| Gln | Gly, Ala, | Thr, Ser, Cys | O<br>‖<br>$-C-NH_2$ | -OH<br>Imidazoyl |
| Arg | Gly, Ala, | Thr, Ser, Cys | $+NH_2$<br>‖<br>$-C - NH_2$ | $-NH_3^+$<br>Imidazolium |

Many enzymes are sufficiently characterized such that the catalytic groups of these enzymes (as defined above) are well known to those skilled in the art. However, for those enzymes which are not so characterized, the catalytic residues can be readily determined.

In this regard, amino acid replacement or chemical modification of catalytic groups (including those directly involved in catalysis and those involved in transition state stabilization) typically cause large disruptions in the catalytic step of the reaction (e.g. often measured by kcat) and little effect on the enzyme substrate dissociation constant (e.g. often measured by Km).

Thus, to determine whether a putative catalytic group is indeed catalytic, one skilled in the art can replace or modify the residue containing that group as described herein. If such substitution or modification abolishes or significantly reduces kcat, but does not substantially effect Km (e.g. increase/ decrease Km by a factor of 50 or preferably 10 or less), the side chain of the residue substituted is a catalytic group.

Structural methods such as x-ray crystallography can also be used to identify potential catalytic groups by their proximity to the site of the substrate chemical bond which becomes altered. Chemical, kinetic and nmr methods can also be useful in identifying catalytic groups by showing a change in their charge or chemical bonding properties during a reaction.

Alternatively, if a particular enzyme is not well characterized but is closely related to an enzyme wherein one or more catalytic groups are already well-defined, the catalytic groups in that enzyme may be identified by determining its equivalent catalytic residues.

Thus, for example, a catalytic residue (amino acid) of a precursor carbonyl hydrolase is equivalent to a residue of B. amyloliquefaciens subtilisin if it is either homologous (i.e., corresponding in position in either primary or tertiary structure) or analagous to a specific residue or portion of that residue in B. amyloliquefaciens subtilisin (i.e., having the same or similar functional capacity to combine, react, or interact chemically).

In order to establish homology to primary structure in the above example, the amino acid sequence of a precursor carbonyl hydrolase is directly compared to the B. amyloliquefaciens subtilisin primary sequence and particularly to a set of residues known to be invariant in all subtilisins for which sequence is known (Figure 3C). After aligning the conserved residues, allowing for necessary insertions and deletions in order to maintain alignment (i.e., avoiding the elimination of conserved residues through arbitrary deletion and insertion), the residues equivalent to the catalytic amino acids (His-64, Asp-32, Ser-221, Asn-155) in the primary sequence of B. amyloliquefaciens subtilisin are defined. Alignment of conserved residues preferably

should conserve 100% of such residues. However, alignment of greater than 75% or as little as 20% of conserved residues is also adequate to define equivalent residues.

For example, in Figure 3A the amino acid sequence of subtilisin from B. amyloliquefaciens B. subtilisin var. I168 and B. lichenformis (carlsbergensis) are aligned to provide the maximum amount of homology between amino acid sequences. A comparison of these sequences shows that there are a number of conserved residues contained in each sequence. These residues are identified in Fig. 3C.

These conserved residues thus may be used to define the corresponding equivalent catalytic amino acid residues of B. amyloliquefaciens subtilisin in other carbonyl hydrolases such as thermitase derived from Thermoactinomyces. These two particular sequences are aligned in Fig. 3B to produce the maximum homology of conserved residues. As can be seen there are a number of insertions and deletions in the thermitase sequence as compared to B. amyloliquefaciens subtilisin. Thus, the equivalent catalytic amino acid of Asn-155 in B. amyloliquefaciens subtilisin in thermitase is the particular lysine shown beneath Asn-155.

Equivalent catalytic residues homologous at the level of tertiary structure for a precursor carbonyl hydrolase whose tertiary structure has been determined by x-ray crystallography, are defined as those for which the atomic coordinates of 2 or more of the main chain atoms of a particular amino acid residue of the precursor carbonyl hydrolase and B. amyloliquefaciens subtilisin (N on N, CA on CA, C on C, and O on O) are within 0.13nm and preferably 0.1nm after alignment. Alignment is achieved after the best model has been oriented and positioned to give the maximum overlap of atomic coordinates of non-hydrogen protein atoms of the carbonyl hydrolase in question to the B. amyloliquefaciens subtilisin. The best model is the crystallographic model giving the lowest R factor for experimental diffraction data at the highest resolution available.

$$\text{R factor} = \frac{\sum_h \left| |Fo(h)| - |Fc(h)| \right|}{\sum_h |Fo(h)|}$$

Equivalent catalytic residues which are functionally analogous to a catalytic residue of B. amyloliquefaciens subtilisin are defined as those amino acids of the precursor carbonyl hydrolases which may adopt a conformation such that they either alter, modify or contribute to catalysis in a manner defined and attributed to a specific catalytic residue of the B. amyloliquefaciens subtilisin as described herein. Further, they are those residues of the precursor carbonyl hydrolase (for which a tertiary structure has been obtained by x-ray crystallography), which occupy an analogous position to the extent that although the main chain atoms of the given residue may not satisfy the criteria of equivalence on the basis of occupying a homologous position, the atomic coordinates of at least two of the side chain atoms of the residue lie with 0.13nm of the corresponding side chain atoms of a catalytic group of B. amyloliquefaciens subtilisin. The three dimensional structures would be aligned as outlined above.

As used herein, a "substrate" refers to a substrate which is reactive with a precursor enzyme. For those enzymes which utilize polypeptides as substrate, the substrate is typically defined by an amino acid sequence which is recognized by the precursor enzyme to bind the substrate therewith. For example, a substrate for trypsin contains the amino acid sequence

$$\begin{matrix} & R & \\ X - & K & - Y \end{matrix}$$

where X is any amino acid, Y is an amino acid except Pro, R is Arg and K is Lys. Subtilisin is broadly specific but will readily cleave a substrate with the sequence FAAY↓AF (at the point designated by the arrow) where F, A, Y are Phe, Ala and Tyr, respectively, and Tyr occupies the P1 position (see Fig. 4). These residues, in general, are those recognized by the enzyme to bind a particular substrate and are referred to herein as a "selected region" of the substrate. Of course, there may be a wide range of polypeptides which are substrates for a particular precursor enzyme. However, each of these substrates will contain a selected region recognized by the precursor enzyme.

In various aspects of the invention, the substrate may be a non-proteinatious molecule such as a

nucleic acid, carbohydrate, a metabolite in a biological pathway or an antibiotic. In the case of nucleic acids and carbohydrates the "substrate" can be similarly defined as for a polypeptide substrate except that these regions are defined not by amino acid sequence but rather by nucleic acid sequence and carbohydrate sequence, respectively. Thus, for example, restriction endonucleases recognize specific DNA sequences and $\alpha$ amylase recognizes the $\alpha(1\rightarrow4)$ glycosidic linkage between glucose molecules in amylose.

In the case of antibiotics, which usually are neither polypeptides, nucleic acids or carbohydrates, there is usually little problem in identifying the substrate. Thus, for example, the substrates for $\beta$ lactamases are penicillins and cephalosporans.

In general, substrates for a wide range of enzymes, including the selected region of such substrates, are known to or may be readily determined by those skilled in the art.

A "modified substrate" is a substrate wherein at least one moiety contained therein is replaced or modified to form a modified moiety which includes the catalytic group replaced or modified in a precursor enzyme or the equivalent of the catalytic group so replaced or modified. The catalytic group contained in the modified substrate is positioned such that upon binding with the mutant enzyme formed by modifying a particular precursor enzyme, the modified substrate provides a modified moiety which then in contact with the mutant enzyme provides the catalytic group replaced in the precursor enzyme or its equivalent. The thus formed enzyme mutant-modified substrate complex is thereby rendered catalytically active.

In the case of precursor enzymes having corresponding polypeptide substrates, the modified substrate is also a polypeptide which contains an amino acid sequence which binds to the mutant enzyme and which contains an amino acid residue within that selected region which has a side chain catalytic group which is the same or equivalent to the amino acid replaced or modified to form the mutant enzyme.

An "equivalent" catalytic group in a modified substrate is one which is capable of reacting, combining or interacting in the same or similar manner as that which was removed from or modified in the precursor enzyme. Equivalent catalytic group refers to a group having the ability to provide a similar or equivalent catalytic role. It is not necessary that an equivalent catalytic group provide equivalent chemical structure. For example, if a catalytic His residue is removed from the enzyme, an equivalent catalytical group from the substrate would be an imidazolyl group which may be donated (but not always or exclusively) by a His side chain. If a catalytic Ser residue is removed from the enzyme an equivalent catalytic group from the substrate may be a hydroxyl group which may be donated by Threonine or Tyrosine side chain. In some cases the equivalent catalytic group may not be identical to the original enzyme group. Thus, an equivalent catalytic group for the Serine-OH may be the Cysteine-SH. See Tables I and II.

Upon binding with the enzyme mutant, the same or equivalent catalytic group in the modified substrate is capable of being positioned such that it is close to the original position of the side chain of the amino acid residue substituted or modified in the precursor enzyme. In this manner the catalytic function of the precursor enzyme can be reestablished when the enzyme mutant binds a modified substrate.

The positioning of the catalytic group or equivalent catalytic group within the selected region of a substrate to form a modified substrate may be achieved by substituting each of the amino acid residues within the selected region with a different amino acid to incorporate the catalytic or equivalent group in the modified substrate at various positions. Such modified substrates can be readily made by the methods disclosed herein and by methods known to those skilled in the art. Thereafter, these modified substrates are contacted with the particular mutant enzyme to determine which, if any, of the modified substrates are reactive with the enzyme mutant.

Alternatively, if the crystal structure of a particular enzyme or enzyme substrate complex is known, model building may be utilized to determine how a modified substrate should be constructed. Such model building may use, for example, a FRODO program (Jones, T.A. (1978) J. Appl. Crystallogr. 11, 268) in conjunction with an Evans and Sutherland PS300 graphics system. For example, the inventors have used such a program and graphic system to construct the stereo view of B. amyloliquefaciens subtilisin shown in Fig. 5 containing a model bound peptide substrate having the sequence L-phe-L-Ala-L-His-L-Tyr-L-Gly-L-Phe representing residues P4-P2' of the substrate.

A two-dimensional representation of the relationship between the subsites involved in subtilisin (S4 through S3') and the substrate residues involved in a substrate binding (P4 through P3') is shown in Fig. 4. Normally, the P2 position in the substrates typically reactive with subtilisin do not require histidine.

The model in Fig. 5 is based upon a 2.0 Å X-ray crystallographic study of product complexes bound to subtilisin. See e.g. Robertus, J.D. et al. (1972) Biochemistry 11, 4293; Poulos, T.L. et al. (1976) J. Biol. Chem. 251, 1097. The catalytic triad (Asp-32, His-64, and Ser-221) is shown with the His P2 side chain from the substrate superimposed upon the catalytic His-64. The distances between the OG of Ser-221 and the corresponding NE2 nitrogens from His-64 and the modeled P2 His side chain are 3.17 Å and 3.17 Å, respectively. The distances between the OD2 of Asp-32 and the corresponding ND1 nitrogens from His-64

and the modeled P2 His side chain are 2.72 Å and 2.72 Å, respectively. The modeled distances between the NE2 and ND1 nitrogens of the histidines are 1.39 Å and 1.35 Å, respectively. The hydrogen bond distances and dihedral angels for the stereo view of the complex of Fig. 4 are given in Table III as subtilisin model S1.

Likewise, the inventors have generated a stereo view of a complex between bovine trypsin and pancreatic trypsin inhibitor (PTI) complex in which the equivalent P2 substrate side chain (Cys-14 in PTI) is replaced by His and superimposed upon His-57 in trypsin. The coordinates for the trypsin/trypsin inhibitor complex were taken from the Brookhaven Protein Data Bank entry 2PTC deposited by R. Huber and J. Deisenhofer, 9/82. See also Deisenhofer, J., et al. (1975) Acta. Crystallogr., Sect. B., 31, 238. The catalytic triad of trypsin (Ser-195, His-57, Asp-102) is shown and the carbonyl carbon of Lys-15 at the P1 position in PTI is labeled. The hydrogen bond distances and dihedral angles for this stereo view in Fig. 9 are given as trypsin model T1 in Table 3.

## TABLE III

**Pertinent bond angles and distances modeled for substrate-assisted catalysis by a His P2 side chain in subtilisin or trypsin as depicted in Figs. 5 and 9, respectively. Dihedral angles for the His side chains are defined by $\chi 1(N-C\alpha-C\beta-C\gamma)$ and $\chi 2(C\alpha-C\beta-C\gamma-C\delta)$. The hydrogen bond angles $(N\epsilon 2(His)-H\delta(ser)-O\gamma(Ser))$ were calculated from the measured $C\beta(Ser)-O\gamma(Ser)-N\epsilon 2(His)$ angle, the $N\epsilon 2(His)-O\gamma(Ser)$**

bond distance and the known Oγ(Ser)-Hδ(Ser) distance (0.96 Å) and the Cβ(Ser)-Oγ(Ser)-Hδ(Ser) bond angle (108.5°) (Weiner, S.J. et al. (1984) J. Am. Chem. Soc. 106, 765). H-bond distances were measured between the catalytic Ser(Oγ) and Asp (Oδ1 and Oδ2) to the Nε2 and Nδ1, respectively, from the enzyme His or the substrate His P2. The distances are given between the enzyme His and the modeled substrate His P2 Nε2 and Nδ1 nitrogens. Model 1 (shown in Fig. 5 and 9 for subtilisin and trypsin, respectively) has the His P2 side chain optimized for H-bond distances between the imidazoyl nitrogen, Nε2 and Nδ1, to the catalytic Ser and Asp, respectively. Model 2 (graphic view not shown) has idealized χ angles for the His P2 side chain.

| | Angles | | | Distances (Å) | | | | |
|---|---|---|---|---|---|---|---|---|
| | Dihedral χ1 | χ2 | H-bond (Ser→His) | Nε2(His) →Oγ(Ser) | Nδ1(His)→ Oδ1(Asp) | or Oδ2(Asp) | Catalytic Nε2/Nε2 | His-His P2 Nδ1/Nδ1 |
| Subtilisin Catalytic His64 (actual) | -167° | 85° | 148° | 3.17 | 3.34 | 2.72 | - | - |
| His P2 side chain Model S1 | -164° | -50° | 149° | 3.17 | 3.55 | 2.72 | 1.39 | 1.35 |
| Model S2 | -180° | -90° | 144° | 3.25 | 3.59 | 3.34 | 0.37 | 1.57 |
| Trypsin Catalytic His57 (actual) | 71° | 85° | 170° | 2.70 | 3.25 | 2.70 | - | - |
| His P2 side chain Model T1 | -155° | -79° | 179° | 2.78 | 4.78 | 3.28 | 0.98 | 2.10 |
| Model T2 | -180° | -90° | 158° | 2.48 | 5.09 | 3.76 | 0.58 | 2.09 |

In general, modified substrates may be naturally occurring substrates containing amino acid sequences which previously were not recognized by the precursor enzyme or other enzymes or may be recombinant substrates. Thus, for example, in the former case the inventors have determined that the subtilisin mutant Cys-24/Ala-64 is reactive with the naturally occurring substrates inhibin (between residues 61 and 80) and ACTH (between residues 1 and 10).

In the latter case, the recombinant substrate is engineered to be reactive with a specific enzyme mutant. Such recombinant substrates include, for example, a fusion polypeptide containing a pro sequence (such as the Trp LE sequence from E. coli) and a desired polypeptide. Such fusion polypeptides are typically generated by recombinant techniques to facilitate the expression and/or secretion of the recombinant polypeptide. However, in many instances, the fused sequence is not cleaved from the desired polypeptide upon secretion or by other known methods (e.g. by relatively nonspecific chemical reactions, such as treatment with CNBr, hydroxylamine, etc.). This problem is overcome by the use of the enzyme mutants of the present invention where the polypeptide sequence of such fusion polypeptides is modified at the juncture of the pro and polypeptide sequences to incorporate a cleavage site which is recognized by the mutant enzyme and which will assist in its own catalysis to produce the desired polypeptide free of the pro sequence. Thus, the invention may be used to engineer a unique cleavage site recognized by a mutant enzyme at the juncture of the pro and polypeptide sequence which does not result in secondary cleavage within the desired polypeptide.

In the case of enzyme mutants which do not act on polypeptide substrates, the modified substrate will consist of a substrate for a precursor enzyme which has been appropriately modified to contain a modified moiety which is catalytic when in contact with the enzyme mutant. Such modified substrates can be designed by substrate modeling as described above using the three-dimensional x-ray crystal structure of a precursor enzyme or enzyme-substrate complex. The construction of such modified substrates, of course, will depend upon the chemical nature of the modified substrate as determined by such modeling and could involve biochemical and/or chemical modification or synthesis of the modified substrate.

In determining how a catalytic group should be replaced or modified in a precursor enzyme, consideration must be given to the modified substrate with which the enzyme is targeted to be reacted with. In general, since the modified substrate will be providing a catalytic group removed from the precursor enzyme, the amino acid residue in the precursor enzyme should be replaced or modified in such a way as to provide space for the modified moiety of the modified substrate. Typically, this requires that the side chain of the precursor amino acid be reduced in volume so that the enzyme mutant can recieve the moiety of the modified substrate.

The mean amino acid volume of amino acids when contained within a protein and the mean side chain volume of such amino acids normalized to a zero side chain volume for glycine are shown in Table IV. As shown in Tables I and II, there are various preferred and alternate amino acids which may be substituted for specific catalytic residues within the active site of a precursor enzyme. In each case, the amino acid being substituted for a catalytic residue has a mean side chain volume which is smaller than the side chain of the catalytic residue replace. In general, the catalytic amino acid residue should be replaced with an amino acid such that the mean side chain volume change upon making the substitution is sufficient to accommodate the catalytic group or equivalent catalytic group of the modified substrate as determined empirically or by modeling studies. Thus, for example, the substitution of His-64 for Ala increases the active site volume by approximately 75 $Å^3$ (101 $Å^3$.-26 $Å^3$). This increase in volume, however, is sufficient to accommodate the histidine at residue P2 in a modified substrate which has a mean side chain volume of 101 $Å^3$.

TABLE IV

| Amino Acid | Chothia(1) Mean Amino Acid Volume in Protein (Å$^3$) | Mean Side Chain Volume(2) (Å$^3$) |
|---|---|---|
| Gly | 66 | 0 |
| Ala | 92 | 26 |
| Ser | 99 | 33 |
| Cys | 118 | 52 |
| Pro | 129 | 63 |
| Thr | 122 | 56 |
| Asp | 125 | 59 |
| Val | 142 | 76 |
| Asn | 135 | 69 |
| Ile | 169 | 103 |
| Glu | 155 | 89 |
| Leu | 168 | 102 |
| Gln | 161 | 95 |
| His | 167 | 101 |
| Met | 171 | 105 |
| Phe | 203 | 137 |
| Lys | 171 | 105 |
| Tyr | 207 | 141 |
| Arg | 202 | 136 |
| Trp | 238 | 172 |

(1) Chothia (1984) Ann. Rev. Biochem. 53, 537
(2) Normalized to zero side chain volume for glycine.

In addition to providing sufficient space for the catalytic group or equivalent catalytic group of the modified substrate, the side chain functionality of the catalytic residue replaced in the precursor enzyme should be altered to facilitate the binding and catalytic activity of the modified substrate. For example, where the side chain of a catalytic amino acid residue in the presursor enzyme contains positively or negatively charged polar groups these amino acids should be replaced or modified to contain side chain which contain non-polar or uncharged polar groups. Such substitutions are summarized in Tables I and II.

"Expression vector" refers to a DNA construct containing a DNA sequence which is operably linked to a suitable control sequence capable of effecting the expression of said DNA in a suitable host. Such control sequences include a promoter to effect transcription, an optional operator sequence to control such transcription, a sequence encoding suitable mRNA ribosome binding sites, and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. In the present specification, "plasmid" and "vector" are sometimes used interchangeably as the plasmid is the most commonly used form of vector at present. However, the invention is intended to include such other forms of expression vectors which serve equivalent functions and which are, or become, known in the art.

The "host cells" used in the present invention generally are procaryotic or eucaryotic hosts which preferably have been manipulated by the methods disclosed in EPO Publication No. 0130756 to render them incapable of secreting enzymatically active endoprotease. A preferred host cell for expressing subtilisin is the Bacillus strain BG2036 which is deficient in enzymatically active neutral protease and alkaline protease (subtilisin). The construction of strain BG2036 is described in detail in EPO Publicatin No. 0130756 and further described by Yang, M.Y., et al. (1984) J. Bacteriol. 160, 15-21. Such host cells are distinguishible from those disclosed in PCT Publication No. 03949 wherein enzymatically inactive mutants of intracellular proteases in E. coli are disclosed. Other host cells for expressing subtilisin include Bacillus subtilis I168 (EPO Publication No. 0130756).

Host cells are transformed or transfected with vectors constructed using recombinant DNA techniques. Such transformed host cells are capable of either replicating vector encoding the enzyme mutants or expressing the desired enzyme mutant. In the case of vectors which encode a pre or prepro form of the enzyme mutant, such mutants, when expressed, are typically secreted from the host cell into the host cell

medium.

"Operably linked" when describing the relationship between two DNA regions simply means that they are functionally related to each other. For example, a presequence is operably linked to a peptide if it functions as a signal sequence, participating in the secretion of the mature form of the protein most probably involving cleavage of the signal sequence. A promoter is operably linked to a coding sequence if it controls the transcription of the sequence; a ribosome binding site is operably linked to a coding sequence if it is positioned so as to permit translation.

The genes encoding the naturally-occurring precursor enzyme may be obtained in accord with the general methods described in EPO Publication No. 0130756 or by other method known to those skilled in the art. As can be seen from the examples disclosed in EPO Publication No. 0130756, the methods generally comprise synthesizing labelled probes having putative sequences encoding regions of the enzyme of interest, preparing genomic libraries from organisims expressing the enzyme, and screening the libraries for the gene of interest by hybridization to the probes. Positively hybridizing clones are then mapped and sequenced.

The cloned enzyme is then used to transform a host cell in order to express the enzyme. The enzyme gene is then ligated into a high copy number plasmid. This plasmid replicates in hosts in the sense that it contains the well-known elements necessary for plasmid replication: a promoter operably linked to the gene in question (which may be supplied as the gene's own homologous promotor if it is recognized, i.e., transcribed, by the host), a transcription termination and polyadenylation region (necessary for stability of the mRHA transcribed by the host from the hydrolase gene in certain eucaryotic host cells) which is exogenous or is supplied by the endogenous terminator region of the hydrolase gene and, desirably, a selection gene such as an antibiotic resistance gene that enables continuous cultural maintenance of plasmid-infected host cells by growth in antibioticcontaining media. High copy number plasmids also contain an origin of replication for the host, thereby enabling large numbers of plasmids to be generated in the cytoplasm without chromosonal limitations. However, it is within the scope herein to integrate multiple copies of the hydrolase gene into host genome. This is facilitated by procaryotic and eucaryotic organisms which ore particularly susceptible to homologous recombination.

Once the precursor enzyme gene has been cloned, a number of modifications are undertaken to enhance the use of the gene beyond synthesis of the naturally-occurring precursor enzyme. Such modifications include the production of recombinant precursor enzymes (as disclosed in EPO Publication No. 0130756) and the production of enzyme mutants.

The following cassette mutagenesis method may be used to facilitate the construction and identification of the enzyme mutants of the present invention although other methods including site-directed mutagenesis may be used. First, the gene encoding the enzyme is obtained and sequenced in whole or in part. Then the sequence is scanned for a point at which it is desired to make a mutation of one or more amino acids in the expressed enzyme. The sequences flanking this point are evaluated for the presence of restriction sites for replacing a short segment of the gene with an oligonucleotide pool which when expressed will encode various mutants. Such restriction sites are preferably unique sites within the gene so as to facilitate the replacement of the gene segment. However, any convenient restriction site which is not overly redundant in the gene may be used, provided the gene fragments generated by restriction digestion can be reassembled in proper sequence. If restriction sites are not present at locations within a convenient distance from the selected point (from 10 to 15 nucleotides), such sites are generated by substituting nucleotides in the gene in such a fashion that neither the reading frame nor the amino acids encoded are changed in the final construction. The task of locating suitable flanking regions and evaluating the needed changes to arrive at two convenient restriction site sequences is made routine by the redundancy of the genetic code, a restriction enzyme map of the gene and the large number of different restriction enzymes. Note that if a convenient flanking restriction site is available, the above method need be used only in connection with the flanking region which does not contain a site.

Mutation of the gene in order to change its sequence to conform to the desired sequence is accomplished by M13 primer extension in accord with generally known methods. Once the gene is cloned, the restriction sites flanking the sequence to be mutated are digested with the cognate restriction enzymes and a plurality of end termini-complementary oligonucleotide cassettes are ligated into the gene. The mutagenesis is enormously simplified by this method because all of the oligonucleotides can be synthesized so as to have the same restriction sites, and no synthetic linkers are necessary to create the restriction sites.

In the disclosed embodiment, subtilisin was chosen as a model to test the concept of substrate-assisted catalysis. In the hydrolysis of peptide bonds by subtilisin, His-64 acts as a catalytic base in the formation of an acyl-enzyme intermediate and as a catalytic acid in the subsequent deacyclation step. Stroud, R.M., et

al. (1975), Proteases and Biological Control (Cold Spring Harbor Laboratory, New York), p. 13; Kraut, J. (1977) Ann. Rev. Biochem. 46, 331.

The catalytic triad of subtilisin is shown in Fig. 2. As can be seen, Ser-221, His-64 and Asp-32 are positioned to facilitate nucleophilic attack by the serine hydoxylate on the carbonyl of the scissile peptide bond. Several hydrogen bonds may also help to stabilize the transition state complex for the tetrahedral substrate intermediate. One hydrogen bond is between aspartate and the positively charged histidine, ND1. Kossiakoff, A.A., et al. (1981) Biochem. 20, 6462-6474. A second hydrogen bond forms between the scissile amide nitrogen of the substrate and the (NE2) proton on the histidine. A third set of hydrogen bonds forms between the enzyme and the oxyanion that is produced from the carbonyl oxygen of the substrate. This latter set of hydrogen bonds is formed differently by the mammalian serine proteases and substilisin. A fourth hydrogen bond appears to exist between the amide nitrogen of the peptide bond between P-1 and P-2 and the carbonyl oxygen of Ser-125. Specifically, x-ray crystallographic studies of chymotrypsin (Henderson, R. (1970) J. Mol. Biol. 54, 341) indicate that two hydrogen bonds form between the substrate oxyanion and two main-chain amide protons from the enzyme (Gly-193 and the catalytic Ser-195). Crystallographic studies of subtilisin (Robertus, et al. (1972) Biochem. 11, 4293-4303; Matthews, et al. (1975) J. Biol. Chem. 250, 7120-7126; Poulos, et al. (1976) J. Biol. Chem. 250, 1097-1103) show that two hydrogen bonds are also formed with the oxyanion; one hydrogen bond donor is from the catalytic Ser-221 main-chain amide while the other is from one of the NE2 protons of the Asd-155 side chain. See Fig. 2.

The model shown in Fig. 5 revealed that the delta and epsilon nitrogens of the histidine in position P2 in the modified substrate can be superimposed within about an angstrom by the corresponding nitrogens of the catalytic His-64 (not shown in Fig. 4). This suggested that if the histidine in the catalytic triad of subtilisin was replaced by an alanine using site directed mutagenisis, a histidine from the substrate may substitue for the missing catalytic group in the mutant enzyme.

Maturation of the primary subtilisin gene product (preprosubtilisin) to subtilisin in B. substilis is believed to be mediated by autoproteolysis that involves trace amounts of active subtilisin (Power, S.D., et al. (1986) Proc. Natl. Acad. Sci USA 83, 3096). The His-64→Ala mutation caused a severe reduction in secretion of mature subtilisin. However, it was possible to process and subsequently purify the inactive Ala-64 mutant by co-culturing B. subtilis cells harboring the Ala-64 mutant gene with B. subtilis cells carrying an active subtilisin gene ("helper").

Stringent precautions were taken to ensure the purification of Ala-64 subtilisin away from "helper" subtilisin and any other contaminating proteases. Firstly, the mutant subtilisin was expressed in the B. subtilis host BG2036 described in EPO Publication No. 0130756, that was deficient in chromosomal copies of the genes for alkaline protease (subtilisin) and neutral protease. Secondly, to minimize "helper" contamination the ratio of "helper" cells to Ala-64 cells in the fermentation culture was adjusted to 1:1,000. Thirdly, a functionally silent Ser-24→Cys mutation that is located on the surface of subtilisin (Wells, J.A., et al. (1986) J. Bio. Chem. 261, 6564) was introduced into the Ala-64 mutant. This accessible cysteine served as an affinity handle for purificiation of the Ala-64 mutant away from the non-cysteine containing "helper" on an activated thiol sepharose column. Finally, the active "helper" subtilisin contained a functionally silent Ala-48→Glu mutation that altered its electrophoretic mobility relative to Cys-24/Ala-64 on native and SDS polyacrylamide gels. After purification, the Cys-24/Ala-64 mutant was judged to be greater than 99% pure by silver stained SDS (Morrissey, J.H. (1981) Anal. Biochem. 11, 307; Laemmli, U.K. (1970) Nature 227, 680) and native polyacrylamide gel electrophoresis. These purification procedures, including the use of a helper subtilisin which is capable of electrophoretic separation from the subtilisin mutant, are not necessarily required to practice the present invention.

## Example 1

### Construction of helper subtilisin containing a functionally silent Ala-48→Glu mutation.

The construction of pS4 is described in detail in EPO Publication No. 0130756. This plasmid is depicted in Fig. 6. pS4 contains 4.5 kb of sequence derived from pBS42 (solid line) and 4.4kb of sequence containing the B. amyloliquefaciens subtilisin gene and flanking sequences (dashed line). pBS42 was constructed as described in EPO Publication No. 0130756 and Band, L. and Henner, D.J. (1984) DNA 3, 17-21. It was digested with BamHI and ligated with Sau3A partially digested chromosomal DNA from B. amyloliquefaciens (ATCC No. 23844) as described in EPO Publication No. 0120756. pS4 was selected from this genomic library. pS4-5, a derivative of pS4 made according to Wells, et al. (1983) Nucleic Acids Res. 11, 7911-7924, was digested with EcoRI and BamHI, and the 1.5 kb EcoRI-BamHI fragment recovered. This fragment was ligated into replicative form M-13 mp9 which had been digested with EcoRI and BamHI

(Sanger, et al., (1980) J. Mol. Biol. 143, 161-178; Messing, et al., (1981) Nucleic Acids Res. 9, 304-321; Messing, J. and Vieira, J. (1982) Gene 19, 269-276). The M-13 mp9 phage ligations, designated M-13 mp9 SUBT, were used to transform E. coli strain JM101 (ATCC 33876) and single stranded phage DNA was prepared from a two mL overnight culture. An oligonucleotide primer was synthesized having the sequence
5'-GTAGCAGGCGGAGAATCCATGGTTCC-3
The primer included the sequence of the subtilisin gene fragment encoding amino acids 44 through 52 except that the codon normally encoding alanine was substituted with the codon GAA encoding glutamate; the serine codon at 49(AGC) was also converted to TCC to introduce a convenient NcoI site.

The primer (about 15 µM) was labelled with [$^{32}$P] by incubation with [$\gamma^{32}$p]-ATP (10 µL in 20 µL reaction) (Amersham 5000 Ci/mmol, 10218) and $T_4$ polynucleotide kinase(10 units) followed by non-radioactive ATP (100 µM) to allow complete phosphorylation of the mutagenesis primer. The kinase was inactivated by heating the phosphorylation mixture to 68° C for 15 minutes.

The primer was hybridized to M-13 mp9 SUBT as modified from Norris, et al., (1983) Nucleic Acids Res. 11, 5103-5112 by combining 5 µL of the labelled mutagenesis primer (~3 µM), ~1 µg M-13 mp9 SUBT template, 1 µL of 1 µM M-13 sequencing primer (17-mer), and 2.5 µL of buffer (0.3 M Tris pH 8, 40 mM $MgCl_2$, 12 mM EDTA, 10 mM DTT, 0.5 mg/ml BSA). The mixture was heated to 68° C for 10 minutes and cooled 10 minutes at room temperature. To the annealing mixture was added 3.6 µL of 0.25 mM dGTP, dCTP, dATP, and dTTP, 1.25 µ of 10 mM ATP, 1 µL ligase (4 units) and 1 µL Klenow (5 units). The primer extension and ligation reaction (total volume 25 µl) proceeded 2 hours at 14° C. The Klenow and ligase were inactivated by heating to 68° C for 20 minutes. The heated reaction mixture was digested with BamHI and EcoRI and an aliquot of the digest was applied to a 6 percent polyacrylamide gel and radioactive fragments were visualized by autoradiography. This showed the [$^{32}$P] mutagenesis primer had indeed been incorporated into the EcoRI-BamHI fragment containing the now mutated subtilisin gene.

The remainder of the digested reaction mixture was diluted to 200 µL with 10 mM Tris, pH 8, containing 1 mM EDTA, extracted once with a 1:1 (v:v) phenol/chloroform mixture, then once with chloroform, and the aqueous phase recovered. 15 µL of 5M ammonium acetate (pH 8) was added along with two volumes of ethanol to precipitate the DNA from the aqueous phase. The DNA was pelleted by centrifugation for five minutes in a microfuge and the supernatant was discarded. 300 µL of 70 percent ethanol was added to wash the DNA pellet, the wash was discarded and the pellet lyophilized.

pBS42 was digested with BamHI and EcoRI and purified on an acrylamide gel to recover the vector. 0.5 µg of the digested vector, 0.1 µg of the above primer mutated EcoRI-BamHI digested subtilisin genonic fragment, 50 µM ATP and 6 units ligase were dissolved in 20 µl of ligation buffer. The ligation went overnight at 14° C. The DNA was transformed into the B. subtilis host BG2036.

## Example 2

### Construction of His-64->Ala Mutant Subtilisin

The B. amyloliquefaciens subtilisin gene on a 1.5kb EcoRI-BamHI fragment (Wells, J.A., et al., (1983) Nucleic Acids Res. 11, 7911-7925) was cloned into M13mp11 (Messing, J. and Vieira, J., (1982) Gene 19, 269-276) to give M13mpllSUBT and single-stranded DNA isolated (Carter, P., et al., (1985) in "Oligonucleotide site-directed mutagenesis in M13" Anglian Biotechnology Limited). The mutation His64->Ala was constructed using the synthetic oligonucleotide HA64

**(5' CAACAACTC*C*G*C*GGAACTCAC 3')**

and the M13SUBT template using a previously described method (Carter, P., et al., (1985) Nucleic Acids Res. 13, 4431-4443). The astrisk in HA64 denote mismatched to the wild-type sequence and underlined is a unique SACII restriction site.

The primer (HA64) was annealed to the single-stranded M13SUBT template extended for 12 hrs. at 4° C with DNA polymerase I (Klenow fragment) in the presence of deoxynucleoside triphosphates and T4 DNA ligase (Carter, P., et al., (1985) Nucleic Acids Res. 13, 4431-4443). The M13 heteroduplex DNA was then transfected directly into the E. coli host BMH 71-18 mutL (Kramer, B., et al., (1984) Cell 38, 879-887). Mutant phage were identified by colony blot hybridization screening as previously described (Carter, P.J., et al., (1984) Cell 38, 835-840).

Putative His64->Ala mutants were verified by dideoxy nucleotide sequencing (Sanger, F., et al., (1977) Proc. Natl. Acad. Sci. USA 77, 5463-5467) as modified by Bankier, A.T. and Barrell, B.G., (1983) in

"Techniques in the life sciences" B5, Nucleic Acids Biochemistry, B508, 1, Elsevier, Ireland and designated M13mp11SUBT-Ala-64. The 1.5kb EcoRI-BamHI fragment from M13mpllSUBT-Ala-64 was isolated and ligated with the 3.7 kb EcoRI-BamHI fragment from the B. subtilis-E. coli shuttle vector pBS42 (Band, L. and Henner, D.J., (1984) DNA 3, 17-21). E. coli MM294 cells (Murray, N.E., et al., (1977) Mol. Gen. Genet. 150, 53) were transformed with the ligation mixture using a $CaCl_2$ procedure (Cohen, S.N., Chang, A.C.Y., and Hsu, L., (1972) Proc. Natl. Acad. Sci. USA 69, 2110-2114). Plasmid DNA was recovered from individual transformants using an alkaline-sodium dodecyl sulfate (SDS) procedure (Birboim, H.C. and Doly, J., (1979) Nucleic Acids Res. 7, 1513-1528 as modified by Burke, J.F. and Ish-Horowicz, D., (1982) Nucleic Acids Res. 10, 3821-3830) to generate pBS42SUBT-Ala-64. The A1a64 mutation was verified by restriction endonuclease digests of the plasmid DNA using the enzymes SacII and BamHI which generate a 0.9kb fragment.

## Example 3

### Construction of the double mutant Ser-24->Cys/His-64->Ala

The double mutant Ser-24->Cys-24/His-64->Ala was constructed from the single mutants pBS42SUBT-Cys-24 (Wells, J.A. and Powers, D.B., (1986) J. Biol. Chem. 261, 6564-6570) and pBS42SUBT-Ala-64 (this document) by a 3-way ligation using the following fragments: 3.7kb EcoRI/BamHI from pBS42, 0.5kb EcoRI/ClaI from pBS42SUBT-Cys-24 and the 1.0kb ClaI/BamHI from pBS42SUBT-Ala-64. The double mutant Cys-24/Ala-64 was identified by restriction endonuclease site markers introduced by the single mutations (His-64->Ala: new SacII site; Ser-24->Ala: Sau3A site removed) and designated pBS42SUBT-Cys-24/Ala-64. The pBS42SUBT-Cys-24/Ala-64 plasmid was introduced into the B. subtilis host BG2036 (Anagostopoluos, C. and Spizizen, J., (1961) J. Bacteriol. 81, 741-746) deficient in alkaline and neutral proteases (Yang, M.Y., et al., (1984) J. Bacteriol. 160, 15-21).

## Example 4

### Co-culturing of Cys-24/Ala-64 and Glu48 mutant subtilisins

Mutant subtilisin genes were expressed in BG2036 by fermentation in shake flasks using 2 X TY media (Miller, J.H., (1972) in "Experiments in Molecular Genetics", Cold Spring Harbor Laboratory, Cold Spring Harbor, NY) containing 12.5 µg/ml chloroamphenicol at 37°C for 18-20 hrs. Co-cultures wre made by diluting Cys-24/Ala-64 cultures 1:100 and Glu-48 cultures 1:100,000 in 2 x TY containing 12.5 µg/ml chloramphenicol and grown at 37°C for 20-24 hrs. with vigorous aeration.

## Example 5

### Purification of Cys-24/Ala-64

Cultures (21) were centrifuged (8,000g, 15 min., 4°C) and 3 volumes of ethanol (-20°C) added to the supernatant. After centrifugation (8,000g, 15 min., 4°C), the pellet was resuspended in 50mM Tris.HCl (pH 8.0), 5mM $CaCl_2$, 10mM dithiothrietol (DTT), 0.1mM phenylmethylsulfonyl fluoride (PMSF). After centrifugation (40,000g, 30 min., 4°C) the supernatant was dialysed against 21 10mM 2-[N-morpholino]ethanesulfonic acid (MES) (pH 6.0), 5mM $CaCl_2$, 10mM DTT, 0.1mM PMSF (S buffer) overnight at 4°C. The dialysate was passed over a 50ml DE52 (Whatman) column and loaded on to 50ml CM Trisacryl (LKB) column. Subtilisin was eluted with a 600ml gradient of S buffer containing 0-100mM Nacl at 1.5 ml/min. Fooled subtilisin containing fractions were dialysed against 21 deaerated 10mM MES (pH 6.0), 5mM $CaCl_2$, 100mM NaCl, 0.1mM PMSF (T buffer). Samples were loaded on to an activated thiol sepharose matrix (Pharmacia) washed extensively with T buffer and then eluted with T buffer containing 20mM DTT. The eluate was concentrated using Centricon 10 microconcentrators (Amicon) and then transferred to 10mM MES (pH 6.0), 5mM $CaCl_2$, 10mM DTT, 0.1mM PMSF (U buffer) by gel filtration using PD10 G25 (Pharmacia) columns. The concentration of subtilisin was determined from the measured absorbance at 280nm ($E_{280}$ 0.1% = 1.17) (Matsubara, H., et al., (1965) J. Biol. Chem. 240, 1125-1130). Aliquots of purified enzyme were flash frozen in liquid nitrogen and then stored at -70 °C.

## Example 6

Preparative native gel electrophoresis

1.5mg Cys-24/Ala-64 subtilisin in U buffer was adjusted to 10mM phenyl boronate, 10% glycerol (v/v) and 0.1% (w/v) methylene blue. The sample was electrophoresed for 24 hrs. at 7W (constant) on a 10% polyacrylamide gel (20cm x 20cm x 0.75cm) with recirculating buffer. The running buffer and gel contained 10mM phenyl boronate, 2mM $CaCl_2$, 5mM DTT 50 mM histidine and 60mM 3-[N-morpholino]-propanesulfonic acid (MOPS). The protein was diffusion-blotted on to nitrocellulose (Hancock, K., and Tsang, V.C.W., (1983 Anal. Biochem. 133, 157-162 as modified by Carter, P., et al., (1986) Proc. Natl. Acad. Sci. USA 83, 1189-1192). Subtilisin was visualized after binding rabbit anti-subtilisin antibody (Power S.D., et al., (1986) Proc. Natl. Acad. Sci. USA 83, 3096-3100) then horse raddish peroxidase conjugated protein A by using the chromogenic substrate 3,3'-diaminobenzidine tetrahydrochloride. Subtilisin containing gel slices were placed in dialysis bags with 6ml running buffer (omitting phenyl boronate) and electroeluted at 10mA (constant) for 20 hrs. at 4°C. Recovered material was concentrated and transferred to U buffer as for column purified enzyme (above).

Example 7

Kinetic analysis of Cys24/Ala64

The kinetic parameters for Cys-24 and Cys-24/Ala-64 were determined against the substrates N-succinyl-L-Phe-L-Ala-L-[X)-L-Phe-*p*-nitroanilide (abbreviated sFAXF-pNA), where X (P2 position) was Ala, Gln, or His (Table I). The kinetic parameters for the Cys-24 enzyme are essentially identical to wild-type subtilisin against these substrates indicating that the Ser24→Cys mutation is kinetically silent. By comparison, the His-64→Ala mutation causes a drop of $\sim 10^6$ fold in kcat/Km against the Ala and Gln P2 substrates. Almost all of the decrease in catalytic efficiency is caused by a decreased $k_{cat}$ term (up to $10^6$ times), although smaller but significant increases appear in Km. Unlike wild-type or Cys-24 subtilisin, the Cys-24/Ala-64 enzyme was completely resistant to inhibition by the active site reagent, phenylmethylsulfonyl-fluoride (PMSF). This suggests the catalytic histidine is critical for stable sulfonylation by PMSF. Although the proportion of functional active sites in Cys-24/Ala-64 enzyme preparations could not be determined directly by such active site labeling, enzyme that was purified by additional native gel electrophoresis (Example 6) had identical kinetic parameters to Cys-24/Ala-64 described in Table V.

The data are consistent with His-64 being extremely important in catalysis (persumably by proton transfer) and only marginally important in substrate binding. However, because we cannot be sure that acylation is rate limiting for the Ala-64 mutant, as it is for the wild-type enzyme (Wells, J.A. (1986) Phil. Trans. R. Soc. Lond. A, 317, 415-423), the relatively small changes in Km may not reflect changes in the enzymesubstrate dissociation constant ($K_s$) but rather a shift in the rate determining step of the reaction (Guttreund, et al. (1956) Biochem J., 63, 656). In any case, the catalytic histidine contributes a factor of about $10^6$ to the total enzymatic rate enhancement (Table V).

The catalytic efficiency of Cys-24 toward the three P2 substrates are all within a factor of five of each other. For the Cys-24/Ala-64 mutant, kcat/Km for the Ala and Gln P2 substrates are essentially the same; however, hydrolysis of the HisP2 substrate is 170 to 210 times more efficient, respectively. Essentially all of the increase in kcat/Km for the His over the Ala and Gln P2 substrates results from the kcat term being larger by a factor of 2,000 and 500, respectively. The larger Km values for the His and Gln P2 substrates compared to Ala may reflect reduced binding affinity resulting from a bulky group at P2. Larger Km values are also observed for the Gln and His substrates for the Cys-24 enzyme. Thus, the drop in kcat/Km caused by the His-64→Ala mutation is partially restored when cleaving a His P2 substrate. The net effect is a marked increase in substrate preference for a His P2 side-chain brought about at the level of catalysis rather than binding. The non-enzymatic hydrolysis rate of the HisP2 substrate is similar to the Ala and Gln P2 substrates (Table V). Thus, the His P2 substrate only becomes functionally active in the context of the catalytic groups provided by the enzyme.

The fact that the catalytic efficiency of the Cys-24/Ala-64 mutant against the His P2 substrate is 5,000 fold below wild-type suggests the His from the substrate P2 functions poorly in catalysis. This may result from the His P2 making poor steric contacts and/or improper alignment of the catalytic triad. Indeed, the model of the His P2 side-chain does not exactly match the catalytic His-64 in that the planes of the histidines from the enzyme and substrate are almost perpendicular to each other (Fig. 5).

Example 8

pH Dependence of Peptide Bond Hydrolysis by $Cys^{24}/Al^{64}$

The pH dependance of $k_{cat}/K_m$ for wild-type subtilisin shows a sigmoidal increase from pH 6 to 8 (Glazer, A.N. (1967), J. Biochem., 242 433) that reflects the titration of the catalytic $His^{64}$ ($pK_a = 7.1 \pm 0.1$). The wild-type pH profile remains relatively flat over the range of 8-10 and declines thereafter (Ottesen, et al. (1970), In Methods of Enzymology (Ed. Perleman, Acad. Press, N.Y., Vol 19, p. 199)).

Fig. 7 shows the pH dependance of hydrolysis of p-nitroanilide peptide substrates by Cys-24/Ala-64 subtilisin. Analysis of Cys-24/Ala-64 against sFAAF-pNA (Fig. 7A) was determined as in Table V except using 100 mM Tris.HCl or 100 mM 3-[cyclohexylamino)-1-propane sulfonic acid (CAPS) buffer. The data was fitted assuming a linear relationship with hydroxide ion concentration (solid lines in Figs. 7A and 7b). Analysis of Cys-24/Ala-64 with sFAHF-pNA (Fig. 7C) was determined as in Table V except using 100mM 3-[N-morpholino) propanesulfonic acid (MOPS) buffer (filled circles) or 100 mM Tris.HCl (open circles) and then normalizing the ionic strength using KC1. The data was fitted to a sigmoid relationship (solid line) using a least-squares fit procedure.

The pH dependence of kcat/Km is markedly different for the Cys-24/Ala-64 enzyme. For the sFAAF-pNA substrate, there is an increase of 15 fold in the kcat/Km between pH 8 to 10 (Fig. 7A). The kcat/Km shows a linear dependence upon hydroxide ion concentration (Fig. 7B) suggesting that a hydroxide ion can act as a catalytic base in the absence of a catalytic histidine side chain. If one were to extrapolate from the increase in kcat/Km as a function of hydroxide concentration ($2 \times 10^4$ $s^{-1}M^{-2}$), to the kcat/Km for Cys-24 against this same Ala P2 substrate ($8 \times 10^5$ $s^1M^1$), then the equivalent concentration of the hydroxide ion would be about 40 M.

In contrast, the kcat/Km for hydrolysis of the sFAHF-pNA by Cys-24/Ala-64 shows a sigmoidal pH dependence between pH 6 and 8 (Fig. 2C) that is similar to wild-type subtilisin. The $pK_a$ of the activity dependent group is 6.8±0.1, and almost all of the pH dependant changes in kcat/Km result from changes in kcat (data not shown). For the sFAHF-pNA substrate, there is not a strong linear increase in kcat/Km with hydroxide above pH 8 as observed for hydrolysis of sFAAF-pNA. These data strongly suggest that the P2 histidine side-chain from the substrate can substitute functionally for the missing catalytic histidine 64.

The data presented in Table V (measured at pH 8.6) underestimate the substrate preference for His over Ala (and Gln) because the kcat/Km for the sFAHF-pNA is maximal at pH 8.0 (Fig. 7C), whereas for the sFAAF-pNA substrate it is significantly lower at pH 8.0 (Fig. 7B). Thus, for Cys-24/Ala-64 at pH 8.0, we estimate the substrate preference is up to -400 times for the His P2 substrate over the corresponding Ala or Gln substrates.

## TABLE V

Kinetic analysis of mutant subtilisin against the substrates, N-succinyl-L-Phe-L-Ala-L-X-L-Phe-ρ-nitroanilide, where X is Ala, Gln, or His. Six hydrolysis assays were performed simultaneously against corresponding substrate blanks in 0.10 M Tris-HCl (pH 8.6), 10 mM DTT at 25±0.1°C using a Kontron unvikon 860 spectrophotometer. Initial reaction rates were determined from the increase in absorbance caused by the release of ρ-nitroaniline group ($\epsilon_M^{410}$=8,480 $M^{-1}$, $cm^{-1}$ (DelMar, E.G., et al. (1979) Anal. Biochem. 99 316)) and fitted by linear regression to an Eadi-Hofstee plot to calculate $V_{max}$ and Km kcat was calculated from $V_{max}$/[enzyme], using the spectrophotometrically determined enzyme concentration (Matsubara, et al. (1965) J. Biol. Chem. 240, 1125). Enzyme concentrations in the assays were about 50 μg/mL for Cys-24/Ala-64 and 1 μg/mL for Cys-24. Standard errors in all determinations were below 20%. Slight variation in the absolute kinetic values has been observed between batches of enzyme, but the relative values among substrates has remained constant.

| Substrate | Cys-24 | | | Cys-24/Ala-64 | | | Non-enzymatic |
|---|---|---|---|---|---|---|---|
| P2 residue | kcat $s^{-1}$ | Km μM | kcat/Km $s^{-1}M^{-1}$ | kcat $s^{-1}$ | Km μM | kcat/Km $s^{-1}M^{-1}$ | hydrolysis rate $s^{-}$ |
| Ala | 8.1 | 10 | $8.0 \times 10^5$ | $8.1 \times 10^{-6}$ | 32 | 0.25 | $1.7 \times 10^{-7}$ |
| Gln | 7.0 | 39 | $1.8 \times 10^5$ | $3.0 \times 10^{-5}$ | 150 | 0.20 | $7.1 \times 10^{-8}$ |
| His | 4.6 | 23 | $2.0 \times 10^5$ | $1.6 \times 10^{-2}$ | 380 | 42 | $7.9 \times 10^{-8}$ |

Several lines of evidence indicate that the activity we attribute to the Cys-24/Ala-64 enzyme is not the result of any other protease contamination. Firstly, the extreme substrate preference for His at the P2 position is unlike wild-type subtilisin or any known Bacillus protease. Secondly, the mutant has Km values which are significantly different from wild-type subtilisin suggesting differences in the energetics of substrate binding and/or catalysis. Thirdly, the mutant is completely resistant to inhibition by PMSF, unlike

other serine proteases. In fact, the kinetic determinations for the Cys-24/Ala-64 mutant are routinely made in the presence of PMSF to exclude any possibility of active "helper" subtilisin (Table V, Fig. 7). Fourthly, the substrate dependent pH profiles are unlike any protease we are aware of. Fifthly, preparations of Cys-24/Ala-64 are extremely pure from other contaminating proteins based by analysis on SDS and native gels (>99%). Finally, the kinetic values determined for Cys-24/Ala-64 that was additionally purified by native gel electrophoresis (Example 6) are essentially the same as these reported in Table V.

Example 9

Hydrolysis of Polypeptide Substrates by Cys-24/Ala-64

To further evaluate the specificity of the Cys-24/Ala-64 mutant in comparison with Cys-24, both enzymes were incubated with a 20 residue fragment of the inhibin B chain at pH 8.0. The choice of the peptide was based upon the presence of two histidines (position 5 and 11) along with 16 different amino acids, and a variety of large hydrophobic amino acids that are preferred amino acids at the P1 position of wild-type subtilisin (Estell, D.A., et al. (1986) Science 233, 659). Fig. 8 shows the hydrolysis of the inhibin peptide substrate TVINHYRMRGHSPFANKLSC by Cys-24/Ala-64 subtilisin. This substrate (100 μg) was digested with 10μg Cys-24/Ala-64 (Fig. 8A) or 0.13 μg Cys-24 (Fig. 8B). Reaction mixtures were in a total volume of 250 μL containing 20 mM Tris.HCl (pH 8.0), 10 mM dithiothreitol, 5% (V/V) dimethyl sulfoxide and 1 mM PMSF (Cys-24/Ala-64 only). After indicated times at 37° C, digestion products (monitored at 214 nm) were eluted from a reverse phase HPLC column (Waters, C18) using a gradient (from left to right) of 0-50% (v/v) acetonitrile in 0.1% (v/v) trifluoroacetic acid.

After a 2-hour incubation with Cys-24/Ala-64 (Fig. 8A), a ~120 fold molar excess of inhibin peptide (peak a) was cleaved to greater than 95% completion into two pieces (peaks b and c). Amino acid composition analysis of these two peptide fragments indicated cleavage had occurred between Tyr-6 and Arg-7, as expected for substrate assisted catalysis by His-5 located at the P2 position from cleavage site. After ten fold longer digestion (20 hr.) a minor third peak appeared (labelled X in Fig. 8A). Analysis showed it to have the same composition as the undigested inhibin peptide. This minor product also appeared in a non-enzymatic blank incubation. No digestion was observed at the second histidine site.

In contrast to the two fragments produced by Cys-24/Ala-64, the Cys-24 enzyme produced at least seven fragments (Fig. 8B) at a similar extent of digestion of starting material (compare 5 min. digestion with Cys-24 to 2 hr. digestion with Cys-24/Ala-64). Although none of these seven fragments were sequenced, the first two produced eluted from the HPLC profile at the same positions as peaks b and c in Fig. 8A. Digestion to 95% completion of the starting peptide by Cys-24 (30 min. incubation, Fig. 8C) produced more than ten different peptide fragments.

Digestion experiments for this and five other peptides are summarized in Table VI. A ten residue fragment of human ACTH was quantitatively cleaved at a single site by Cys-24/Ala-64. Amino acid composition analysis of the two digetion products confirmed that the cleavage had occurred with a His residue at the P2 position of the substrate as expected. However digestion of this peptide with Cys-24 also gave specific cleavage at this position (not shown). This probably resulted because Phe provides a very favourable P1 residue, and the two short peptides liberated do not provide effective substrates for subtilisin. The four other peptides tested (three containing His and one which did not) were not cleaved by Cys-24/Ala-64 but were cleaved at several sites by the Cys-24 mutant (not shown).

## TABLE VI

Digestion of peptide substrates by Cys-24/Ala-64 subtilisin. Various synthetic peptides (200 μg) shown were digested with Cys-24/Ala-64 (10 μg) in 20 mM Tris-HCl (pH 8.0), 10 mM DTT, 5% (v/v) dimethyl sulfoxide, 1 mM PMSF (250 μL total volulme) for 20 hr. at 37°C. Digestion products were analyzed by reverse phase HPLC as described in Fig. 3. Digestion products recovered by HPLC were hydrolyzed for 24 hr. in 6 N HCl, 1% (v/v) phenol before amino acid analysis using nor-leucine as an internal standard. The Cys residues in bovine insulin A and B chains were oxidized to $CysSO_3H$. Sequences are designated by the single letter amino acid code.

| Peptide Source | Sequence | Cleavage Peptides with Cys-24/Ala-64 |
|---|---|---|
| Inhibin β-chain Residues 61-80 | TVINHYRMRGHSPFANLKSC | TVINHY + RMRGHSPFANLKSC |
| ACTH Residues 1-10 | SYSMEHFRWG | SYSMEHF + RWG |
| Ubiquitin | CKESTLHLVLRLRGG | Not cleaved |
| Peptide C | GYEHFENLRRRAASFQGKY | Not cleaved |
| Bovine insulin B chain (oxidized) | FVNQHLCGSHLVEALYLVCGERGFFYTPKA | Not cleaved |
| Bovine insulin A chain (oxidized) | GIVEQCCASVCSLYQLENYCN | Not cleaved |

These experiments establish much about the activity, specificity, and utility of the Cys-24/Ala-64 mutant. In addition to ρ-nitroanilide substrates, the enzyme is capable of cleaving normal peptide bonds. Unlike the Cys-24 enzyme, the specificity of Cys-24/Ala-64 appears to be limited to sites containing a histidine side chain located at the P2 position of the cleavage site. Furthermore, additional specificity determinants are required because not all His P2 sites are cleaved. We believe that this reflects the normal specificity determinants in the wild-type enzyme. Peptide substrates were chosen to have His followed by a large hydrophobic amino acid which is preferred for the P1 site in subtilisin (Estell, D.A. et al. (1986) Sci. 233, 659; Phillip, M. et al. (1983) Mol. Cell Biochem. 51, 5; Svendsen, I. (1976) Carlsberg Res. Commun. 41, 237). Little is known about P1' specificity but the absence of cleavage at the other His sites may reflect the presence of a negatively charged (Glu or Cys $SO_3H$), a β-branched (Val) or a proline all of which are very

poorly hydrolyzed P1 amino acids (Estell, D.A. et al. (1986) Sci. 233, 659). All literature references are expressly incorporated herein by reference.

Having described the preferred embodiments of the present invention, it will appear to those ordinarily skilled in the art that various modifications may be made to the disclosed embodiments and that such modifications are intended to be within the scope of the present invention.

**Claims**

**1.** An enzyme mutant, not found in nature, said enzyme mutant being derived by the replacement or modification, in a precursor enzyme, of at least one catalytic group of an amino acid residue which when in contact with a selected region of a polypeptide substrate functions catalytically therewith, to form an enzyme mutant which is relatively inactive catalytically with said polypeptide substrate as compared to said enzyme mutant's catalytic activity with at least one modified substrate, said modified substrate being formed by replacing or modifying a moiety in said selected region to form a modified moiety which includes said one catalytic group or its equivalent.

**2.** The enzyme mutant of claim 1 wherein said one catalytic group in said precursor enzyme is substituted with a second group having a volume which is less than the volume of said one catalytic group.

**3.** The enzyme mutant of claim 1 wherein said replacement is of a catalytic amino acid residue in said precursor enzyme with a different amino acid residue, said catalytic amino acid residue is selected from the group consisting of His, Lys, Ser, Thr, Cys, Asp, Glu, Tyr Met, Phe and Trp and wherein said different amino acid is selected from the preferred or alternate amino acid residues of Table I herein.

**4.** The enzyme mutant of claim 3 wherein said modified substrate contains a modified moiety including a catalytic group or equivalent catalytic group of Table I herein corresponding to said replaced catalytic amino acid residue in said precursor enzyme.

**5.** The enzyme mutant of claim 1 wherein said precursor enzyme is selected from the group consisting of oxido-reductases, transferases, hydrolases, lyases, isomerases and ligases.

**6.** The enzyme mutant of claim 5 wherein said precursor enzyme is a hydrolase comprising a carbonyl hydrolase.

**7.** The enzyme mutant of claim 6 wherein said carbonyl hydrolase is subtilisin.

**8.** The enzyme mutant of claim 7 wherein said replaced or modified amino acid residue in said subtilisin is His-64 in B. amyloliquefaciens subtilisin.

**9.** The enzyme mutant of claim 8 wherein said His-64 is replaced by Ala.

**10.** The enzyme mutant of claim 8 wherein said modified substrate contains a modified moiety located at residue P2 of said modified substrate.

**11.** The enzyme mutant of claim 10 wherein said modified substrate is formed by replacing said moiety at position P2 with histidine.

**12.** A DNA sequence encoding the enzyme mutant of claim 1.

**13.** An expression vector containing the DNA of claim 12.

**14.** Host cells transformed with the expression vector of claim 13.

**15.** A catalytically active enzyme-substrate complex comprising an enzyme mutant in contact with a modified substrate, wherein said enzyme mutant is not found in nature and is derived by the replacement or modification, in a precursor enzyme, of at least one catalytic group of an amino acid residue which when in contact with a selected region of a substrate functions catalytically therewith, to form said enzyme mutant which is relatively inactive catalytically with said substrate as compared to

said enzyme mutant's catalytic activity with at least one said modified substrate, said modified substrate being formed by replacing or modifying a moiety in said selected region to form a modified moiety which includes said one functional group or its equivalent.

**16.** The enzyme substrate complex of claim 15 wherein said one catalytic group in said precursor enzyme is substituted with a second group having a volume which is less than the volume of said one catalytic group.

**17.** The enzyme-substrate complex of claim 15 wherein said replacement is of a catalytic amino acid residue in said precursor enzyme with a different amino acid, said catalytic amino acid residue is selected from the group consisting of His, Lys, Ser, Thr, Cys, Asp, Glu, Tyr, Met, Phe, Trp, Asn, Gln and Arg and wherein said different amino acid residue is selected from preferred or alternate amino acid residues of Tables I or II herein.

**18.** The enzyme-substrate complex of claim 15 wherein said modified substrate contains a modified moiety including a catalytic group or equivalent catalytic group of Tables I or II herein corresponding to said replaced catalytic amino acid residue in said precursor enzyme.

**19.** The enzyme-substrate complex of claim 15 wherein said precursor enzyme is selected from the group consisting of oxido-reductases, transferases, hydrolases, lyases, isomerases and ligases.

**20.** The enzyme-substrate complex of claim 19 wherein said precursor enzyme is a hydrolase comprising a carbonyl hydrolase.

**21.** The enzyme-substrate complex of claim 20 wherein said carbonyl hydrolase is subtilisin.

**22.** The enzyme-substrate complex of claim 21 wherein said replaced or modified amino acid residue in said subtilisin is His-64 in B. amyloliquefaciens subtilisin.

**23.** The enzyme-substrate complex of claim 22 wherein said His-64 is replaced by Ala.

**24.** The enzyme-substrate complex of claim 22 wherein said modified substrate contains a modified moiety located at residue P2 of said modified substrate.

**25.** The enzyme-substrate complex of claim 24 wherein said modified substrate is formed by replacing said moiety at position P2 with histidine.

**26.** A process comprising contacting an enzyme mutant and a modified substrate to produce the substrate assisted catalysis of said modified substrate, wherein said enzyme mutant is not found in nature and is derived by the replacement or modification, in a precursor enzyme, of at least one catalytic group of an amino acid residue which when in contact with a selected region of a substrate functions catalytically therewith, to form said enzyme mutant which is relatively inactive with said substrate as compared to said enzyme mutant's catalytic activity with at least said modified substrate, said modified substrate being formed by replacing or modifying a moiety in said selected region to form a modified moiety which includes said one catalytic functional group or its equivalent.

**27.** The process of claim 26 wherein said one catalytic group in said precursor enzyme is substituted with a second group having a volume which is less than the volume of said one catalytic group.

**28.** The process of claim 26 wherein said replacement is of a catalytic amino acid residue in said precursor enzyme with a different amino acid, said catalytic amino acid residue is selected from the group consisting of His, Lys, Ser, Thr, Cys, Asp, Glu, Tyr, Met, Phe, Trp, Asn, Gln and Arg and wherein said different amino acid residue is selected from preferred or alternate amino acid residues of Tables I or II herein.

**29.** The process of claim 28 wherein said modified substrate contains a modified moiety including a catalytic group or equivalent catalytic group of Tables I or II herein corresponding to said replaced catalytic amino acid residue in said precursor enzyme.

**30.** The process of claim 27 wherein said precursor enzyme is selected from the group consisting of oxidoreductases, transferases, hydrolases, lyases, isomerases and ligases.

**31.** The process of claim 30 wherein said precursor enzyme is a hydrolase comprising a carbonyl hydrolase.

**32.** The process of claim 31 wherein said carbonyl hydrolase is subtilisin.

**33.** The process of claim 32 wherein said replaced or modified amino acid residue in said subtilisin is His-64 in B. amyloliquefaciens subtilisin.

**34.** The process of claim 33 wherein said His-64 is replaced by Ala.

**35.** The process of claim 33 wherein said modified substrate contains a modified moiety located at residue P2 of said modified substrate.

**36.** The process of claim 35 wherein said modified substrate is formed by replacing said moiety at position P2 with histidine.

**Patentansprüche**

**1.** Enzymmutant, der nicht in der Natur vorkommt, wobei der genannte Enzymmutant durch das Ersetzen oder die Modifikation von zumindest einer katalytischen Gruppe eines Aminosäurerests in einem Vorläuferenzym hergeleitet ist, der, wenn er mit einem ausgewählten Bereich eines Polypeptidsubstrats in Kontakt steht, katalytisch damit wirkt, um einen Enzymmutanten zu bilden, der mit dem genannten Polypeptidsubstrat im Vergleich zur katalytischen Wirksamkeit des genannten Enzymmutanten mit zumindest einem modifizierten Substrat katalytisch relativ inaktiv ist, wobei das genannte modifizierte Substrat durch Ersetzen oder Modifizieren einer Gruppe im genannen ausgewählten Bereich gebildet wird, um eine modifizierte Gruppe zu bilden, welche die genannte eine katalytische Gruppe oder ihr Äquivalent enthält.

**2.** Enzymmutant nach Anspruch 1, worin die genannte eine katalytische Gruppe im genannten Vorläuferenzym mit einer zweiten Gruppe substituiert ist, die ein Volumen aufweist, das geringer als das Volumen der genannten einen katalytischen Gruppe ist.

**3.** Enzymmutant nach Anspruch 1, worin das genannte Ersetzen einen katalytischen Aminosäurerest im genannten Vorläuferenzym durch einen anderen Aminosäurerest betrifft, wobei der genannte katalytische Aminosäurerest aus der Gruppe ausgewählt ist, die aus His, Lys, Ser, Thr, Cys, Asp, Glu, Tyr, Met, Phe und Trp besteht, und worin die genannte andere Aminosäure aus den bevorzugten oder alternativen Aminosäureresten von Tabelle I hierin ausgewählt ist.

**4.** Enzymmutant nach Anspruch 3, worin das genannte modifizierte Substrat eine modifizierte Gruppe enthält, die eine katalytische Gruppe oder äquivalente katalvtische Gruppe von Tabelle I hierin einschließt, die dem genannten ersetzten katalytischen Aminosäurerest im genannten Vorläuferenz entspricht.

**5.** Enzymmutant nach Anspruch 1, worin das genannte Vorläuferenzym aus der Gruppe ausgewählt ist, die aus Oxido-Reduktasen, Transferasen, Hydrolasen, Lyasen, Isomerasen und Ligasen besteht.

**6.** Enzymmutant nach Anspruch 5, worin das genannte Vorläuferenzym eine Hydrolase ist, die eine Carbonylhydrolase umfaßt.

**7.** Enzymmutant nach Anspruch 6, worin die genannte Carbonylhydrolase Subtilisin ist.

**8.** Enzymmutant nach Anspruch 7, worin der genannte ersetzte oder modifizierte Aminosäurerest im genannten Subtilisin His-64 in B.amyloliquefaciens Subtilisin ist.

**9.** Enzymmutant nach Anspruch 8, worin das genannte His-64 durch Ala ersetzt ist.

**10.** Enzymmutant nach Anspruch 8, worin das genannte modifizierte Substrat eine modifizierte Gruppe enthält, die an Rest P2 des genannten modifizierten Substrats angeordnet ist.

**11.** Enzymmutant nach Anspruch 10, worin das genannte modifizierte Substrat durch Ersetzen der genannten Gruppe an Position P2 durch Histidin gebildet ist.

**12.** DNA-Sequenz, die für den Enzymmutanten nach Anspruch 1 kodiert.

**13.** Expressionsvektor, der die DNA nach Anspruch 12 enthält.

**14.** Mit dem Expressionsvektor nach Anspruch 13 transformierte Wirtszellen.

**15.** Katalytisch aktiver Enzymsubstratkomplex, der einen Enzymmutanten in Kontakt mit einem modifizierten Substrat umfaßt, worin der genannte Enzymmutant nicht in der Natur vorkommt und durch das Ersetzen oder die Modifikation von zumindest einer katalytischen Gruppe eines Aminosäurerests in einem Vorläuferenz hergeleitet ist, der, wenn er mit einem ausgewählten Bereich eines Substrats in Kontakt steht, katalytisch damit wirkt, um den genannten Enzymmutanten zu bilden, der mit dem genannten Substrat im Vergleich zur katalytischen Wirksamkeit des genannten Enzymmutanten mit zumindest einem genannten modifizierten Substrat katalvtisch relativ inaktiv ist, wobei das genannte modifizierte Substrat durch das Ersetzen oder Modifizieren einer Gruppe im genannten ausgewählten Bereich gebildet ist, um eine modifizierte Gruppe zu bilden, welche die genannte eine funktionelle Gruppe oder ihr Äquivalent enthält.

**16.** Enzymsubstratkomplex nach Anspruch 15, worin die genannte eine katalytische Gruppe im genannten Vorläuferenzym durch eine zweite Gruppe substituiert ist, die ein Volumen aufweist, das geringer als das Volumen der genannten einen katalytischen Gruppe ist.

**17.** Enzymsubstratkomplex nach Anspruch 15, worin das genannte Ersetzen einen katalytischen Aminosäurerest im genannten Vorläuferenzym mit einer anderen Aminosäure betrifft, wobei der genannte katalytische Aminosäurerest aus der Gruppe ausgewählt ist, die aus His, Lys, Ser, Thr, Cys, Asp, Glu, Tyr, Met, Phe, Trp, Asn, Gln und Arg besteht, und worin der genannte andere Aminosäurerest aus bevorzugten oder alternativen Aminosäureresten von Tabelle I oder II hierin ausgewählt ist.

**18.** Enzymsubstratkomplex nach Anspruch 15, worin das genannte modifizierte Substrat eine modifizierte Gruppe enthält, die eine katalytische Gruppe oder eine äquivalente katalytische Gruppe von Tabelle I oder II hierin einschließt, die dem genannten ersetzten katalytischen Aminosäurerest im genannten Vorläuferenzym entspricht.

**19.** Enzymsubstratkomplex nach Anspruch 15, worin das genannte Vorläuferenzym aus der Gruppe ausgewählt ist, die aus Oxido-Reduktasen, Transferasen, Hydrolasen, Lyasen, Isomerasen und Ligasen besteht.

**20.** Enzymsubstratkomplex nach Anspruch 19, worin das genannte Vorläuferenzym eine Hydrolase ist, die eine Carbonylhydrolase umfaßt.

**21.** Enzymsubstratkomplex nach Anspruch 20, worin die genannte Carbonylhydrolase Subtilisin ist.

**22.** Enzymsubstratkomplex nach Anspruch 21, worin der genannte ersetzte oder modifizierte Aminosäurerest im genannten Subtilisin His-64 in B. amyloliquefaciens Subtilisin ist.

**23.** Enzymsubstratkomplex nach Anspruch 22, worin das genannte His-64 durch Ala ersetzt ist.

**24.** Enzymsubstratkomplex nach Anspruch 22, worin das genannte modifizierte Substrat eine modifizierte Gruppe enthält, die an Rest P2 des genannten modifizierten Substrats angeordnet ist.

**25.** Enzymsubstratkomplex nach Anspruch 24, worin das genannte modifizierte Substrat durch Ersetzen der genannten Gruppe an Position P2 durch Histidin gebildet ist.

26. Verfahren, umfassend das In-Kontakt-bringen eines Enzymutanten und eines modifizierten Substrats, um die substratunterstützte Katalyse des genannten modifizierten Substrats zu erzeugen, worin der genannte Enzymmutant nicht in der Natur vorkommt und durch das Ersetzen oder die Modifikation von zumindest einer katalytischen Gruppe eines Aminosäurerests in einem Vorläuferenzym hergeleitet ist, der, wenn er mit einem ausgewählten Bereich eines Substrats in Kontakt steht, katalytisch damit wirkt, um den genannten Enzymmutanten zu bilden, der mit dem genannten Substrat im Vergleich zur katalytischen Wirksamkeit des genannten Enzymmutanten mit zumindest dem genannten modifizierten Substrat relativ inaktiv ist, wobei das genannte modifizierte Substrat durch Ersetzen oder Modifizieren einer Gruppe im genannten ausgewählten Bereich gebildet wird, um eine modifizierte Gruppe zu bilden, welche die genannte eine katalytische funktionelle Gruppe oder ihr Äquivalent enthält.

27. Verfahren nach Anspruch 26, worin die genannte eine katalytische Gruppe im genannten Vorläuferenzym durch eine zweite Gruppe substituiert wird, die ein Volumen aufweist, das geringer als das Volumen der genannten einen katalytischen Gruppe ist.

28. Verfahren nach Anspruch 26, worin das genannte Ersetzen einen katalytischen Aminosäurerest im genannten Vorläuferenzym durch eine andere Aminosäure betrifft, wobei der genannte katalytische Aminosäurerest aus der Gruppe ausgewählt ist, die aus His, Lys, Ser, Thr, Cys, Asp, Glu, Tyr, Met, Phe, Trp, Asn, Gln und Arg besteht, und worin der genannte andere Aminosäurerest aus bevorzugten oder alternativen Aminosäureresten von Tabelle I oder II hierin ausgewählt ist.

29. Verfahren nach Anspruch 28, worin das genannte modifizierte Substrat eine modifizierte Gruppe enthält, die eine katalytische Gruppe oder äquivalente katalytische Gruppe von Tabelle I oder II hierin enthält, die dem genannten ersetzten katalytischen Aminosäurerest im genannten Vorläuferenzym entspricht.

30. Verfahren nach Anspruch 27, worin das genannte Vorläuferenzym aus der Gruppe ausgewählt ist, die aus Oxido-Reduktasen, Transferasen, Hydrolasen, Lyasen, Isomerasen und Ligasen besteht.

31. Verfahren nach Anspruch 30, worin das genannte Vorläuferenzym eine Hydrolase ist, die eine Carbonylhydrolase umfaßt.

32. Verfahren nach Anspruch 31, worin die genannte Carbonylhydrolase Subtilisin ist.

33. Verfahren nach Anspruch 32, worin der genannte ersetzte oder modifizierte Aminosäurerest im genannten Subtilisin His-64 in B. amyloliquefaciens Subtilisin ist.

34. Verfahren nach Anspruch 33, worin das genannte His-64 durch Ala ersetzt wird.

35. Verfahren nach Anspruch 33, worin das genannte modifizierte Substrat eine modifizierte Gruppe enthält, die an Rest P2 des genannten modifizierten Substrats angeordnet ist.

36. Verfahren nach Anspruch 35, worin das genannte modifizierte Substrat durch Ersetzen der genannten Gruppe an Position P2 durch Histidin gebildet wird.

**Revendications**

1. Mutant d'enzyme, non présent dans la nature, ledit mutant d'enzyme étant obtenu par remplacement ou modification, dans un enzyme précurseur, d'au moins un groupe catalytique d'un résidu d'aminoacide qui, lorsqu'il est en contact avec une région choisie d'un substrat polypeptidique, agit catalytiqueqment avec cette région pour former un mutant d'enzyme qui est relativement inactif du point de vue catalytique avec ledit substrat polypeptidique, comparativement à l'activité catalytique dudit mutant d'enzyme avec au moins un substrat modifié, ledit substrat modifié étant formé par remplacement ou modification d'un groupement dans ladite région choisie pour former un groupement modifié qui comprend ledit groupe catalytique ou son équivalent.

2. Mutant d'enzyme suivant la revendication 1, dans lequel le groupe catalytique dans l'enzyme précurseur est substitué avec un second groupe ayant un volume qui est inférieur au volume dudit groupe

catalytique.

**3.** Mutant d'enzyme suivant la revendication 1, dans lequel le remplacement est le remplacement d'un résidu d'amino-acide catalytique dans l'enzyme précurseur par un résidu d'amino-acide différent, ledit résidu d'amino-acide catalytique étant choisi dans le groupe comprenant His, Lys, Ser, Thr, Cys, Asp, Glu, Tyr, Met, Phe et Trp, et dans lequel l'amino-acide différent est choisi entre les résidus d'aminoacides préférés ou différents mentionnés sur le Tableau I de la présente invention.

**4.** Mutant d'enzyme suivant la revendication 3, dans lequel le substrat modifié contient un groupement modifié comprenant un groupe catalytique ou un groupe catalytique équivalent mentionné sur le Tableau I de la présente invention, correspondant au résidu d'amino-acide catalytique remplacé dans l'enzyme précurseur.

**5.** Mutant d'enzyme suivant la revendication 1, dans lequel l'enzyme précurseur est choisi dans le groupe comprenant des oxydo-réductases, des transférases, des hydrolases, des lyases, des isomérases et des ligases.

**6.** Mutant d'enzyme suivant la revendication 5, dans lequel l'enzyme précurseur est une hydrolase, consistant en une carbonyl-hydrolase.

**7.** Mutant d'enzyme suivant la revendication 6, dans lequel la carbonylhydrolase est la subtilisine.

**8.** Mutant d'enzyme suivant la revendication 7, dans lequel le résidu d'amino-acide remplacé ou modifié dans la subtilisine est His-64 dans la subtilisine de B. amyloliquefaciens.

**9.** Mutant d'enzyme suivant la revendication 8, dans lequel la His-64 est remplacée par Ala.

**10.** Mutant d'enzyme suivant la revendication 8, dans lequel le substrat modifié contient un groupement modifié situé au niveau du résidu P2 dudit substrat modifié.

**11.** Mutant d'enzyme suivant la revendication 10, dans lequel le substrat modifié est formé par remplacement du groupement en position P2 par l'histidine.

**12.** Séquence d'ADN codant pour le mutant d'enzyme suivant la revendication 1.

**13.** Vecteur d'expression contenant l'ADN suivant la revendication 12.

**14.** Cellules hôtes transformées avec le vecteur d'expression suivant la revendication 13.

**15.** Complexe enzyme-substrat catalytiquement actif, comprenant un mutant d'enzyme en contact avec un substrat modifié, dans lequel ledit mutant d'enzyme n'existe pas dans la nature et provient du remplacement ou de la modification, dans un emzyme précurseur, d'au moins un groupe catalytique d'un résidu d'amino-acide qui, lorsqu'il est en contact avec une région choisie d'un substrat, agit catalytiquement avec ce substrat, pour former ledit mutant d'enzyme qui est relativement inactif du point de vue catalytique avec ledit substrat comparativement à l'activité catalytique dudit mutant d'enzyme avec au moins ledit substrat modifié, ledit substrat modifié étant formé par remplacement ou modification d'un groupement dans ladite région choisie pour former un groupement modifié qui comprend ledit groupe fonctionnel ou son équivalent.

**16.** Complexe enzyme-substrat suivant la revendication 15, dans lequel le groupe catalytique dans l'enzyme précurseur est substitué avec un second groupe ayant un volume qui est inférieur au volume dudit groupe catalytique.

**17.** Complexe enzyme-substrat suivant la revendication 15, dans lequel le remplacement consiste en le remplacement d'un résidu d'amino-acide catalytique dans l'enzyme précurseur par un amino-acide différent, ledit résidu d'amino-acide catalytique étant choisi dans le groupe comprenant His, Lys, Ser, Thr, Cys, Asp, Glu, Tyr, Met, Phe, Trp, Asn, Gln et Arg, et dans lequel le résidu d'amino-acide différent est choisi entre les résidus d'amino-acides préférés ou de remplacement figurant sur les Tableaux I et

II de la présente invention.

**18.** Complexe enzyme-substrat suivant la revendication 15, dans lequel le substrat modifié contient un groupement modifié renfermant un groupe catalytique ou groupe catalytique équivalent figurant sur les Tableaux I et II de la présente invention correspondant au résidu d'amino-acide catalytique remplacé dans l'enzyme précurseur.

**19.** Complexe enzyme-substrat suivant la revendication 15, dans lequel l'enzyme précurseur est choisi dans le groupe comprenant des oxydoréductases, des transférases, des hydrolases, des lyases, des isomérases et des ligases.

**20.** Complexe enzyme-substrat suivant la revendication 19, dans lequel l'enzyme précurseur est une hydrolase, consistant en une carbonyl-hydrolase.

**21.** Complexe enzyme-substrat suivant la revendication 20, dans lequel la carbonyl-hydrolase est la subtilisine.

**22.** Complexe enzyme-substrat suivant la revendication 21, dans lequel le résidu d'amino-acide remplacé ou modifié dans la subtilisine est His-64, dans la subtilisine de B. amyloliquefaciens.

**23.** Complexe enzyme-substrat suivant la revendication 22, dans lequel la His-64 est remplacée par Ala.

**24.** Complexe enzyme-substrat suivant la revendication 22, dans lequel le substrat modifié contient un groupement modifié situé au niveau du résidu P2 dudit substrat modifié.

**25.** Complexe enzyme-substrat suivant la revendication 24, dans lequel le substrat modifié est formé par remplacement du groupement en position P2 par l'histidine.

**26.** Procédé consistant à mettre en contact un mutant d'enzyme et un substrat modifié pour provoquer la catalyse assistée par le substrat dudit substrat modifié, dans lequel ledit mutant d'enzyme n'existe pas dans la nature et est obtenu par remplacement ou modification, dans un enzyme précurseur, d'au moins un groupe catalytique d'un résidu d'amino-acide qui, lorsqu'il est contact avec une région choisie d'un substrat, agit catalytiquement avec ce substrat, pour former ledit mutant d'enzyme qui est relativement inactif avec ledit substrat comparativement à l'activité catalytique dudit mutant d'enzyme avec au moins ledit substrat modifié, ledit substrat modifié étant formé par remplacement ou modification d'un groupement dans ladite région choisie pour former un groupement modifié qui comprend ledit groupe fonctionnel catalytique ou son équivalent.

**27.** Procédé suivant la revendication 26, dans lequel le groupe catalytique dans l'enzyme précurseur est substitué avec un second groupe ayant un volume qui est inférieur au volume dudit groupe catalytique.

**28.** Procédé suivant la revendication 26, dans lequel le remplacement consiste en le remplacement d'un résidu d'amino-acide catalytique dans l'enzyme précurseur avec un amino-acide différent, ledit résidu d'amino-acide catalytique étant choisi dans le groupe comprenant His, Lys, Ser, Thr, Cys, Asp, Glu, Tyr, Met, Phe, Trp, Asn, Gln et Arg, et dans lequel ledit résidu d'amino-acide différent est choisi entre des résidus d'amino-acides préférés ou de remplacement figurant sur les Tableaux I et II de la présente invention.

**29.** Procédé suivant la revendication 28, dans lequel le substrat modifié contient un groupement modifié comprenant un groupe catalytique ou groupe catalytique équivalent figurant sur les Tableaux I et II de la présente invention correspondant au résidu d'amino-acide catalytique remplacé dans l'enzyme précurseur.

**30.** Procédé suivant la revendication 27, dans lequel l'enzyme précurseur est choisi dans le groupe comprenant des oxydo-réductase, des transférases, des hydrolases, des lyases, des isomérases et des ligases.

**31.** Procédé suivant la revendication 30, dans lequel l'enzyme précurseur est une hydrolase, consistant en

une carbonyl-hydrolase.

**32.** Procédé suivant la revendication 31, dans lequel la carbonyl-hydrolase est la subtilisine.

**33.** Procédé suivant la revendication 32, dans lequel le résidu d'amino-acide remplacé ou modifié dans la subtilisine est His-64 dans la subtilisine de B. amyloliquefaciens

**34.** Procédé suivant la revendication 33, dans lequel la His-64 est remplacée par Ala.

**35.** Procédé suivant la revendication 33, dans lequel le substrat modifié contient un groupement modifié situé au niveau du résidu P2 dudit substrat modifié.

**36.** Procédé suivant la revendication 35, dans lequel le substrat modifié est formé par remplacement du groupement en position P2 par l'histidine.

FIG. 1a.

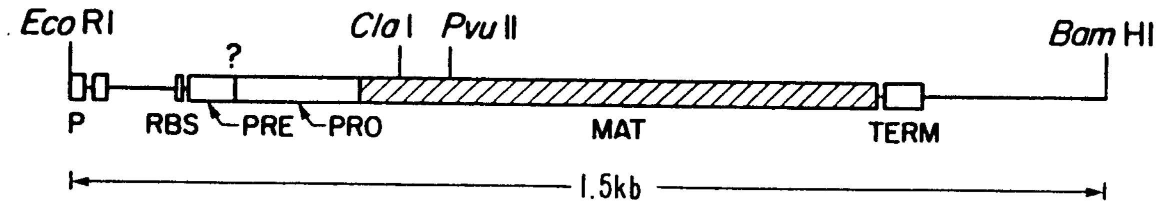


FIG. 1b. (top)

(5) P (3) (4) RBS

```
                                                                                              -107
                                                                                              fMet
1  GGTCTACTAAAATATTATTCCATACTATACAATTAATACACAGAATAATCTGTCTATTGGTTATTCTGCAAATGAAAAAAAGGAGAGGATAAAGA  GTG

                         -100             PRE                    -90
   Arg Gly Lys Lys Val Trp Ile Ser Leu Leu Phe Ala Leu Ala Leu Ile Phe Thr Met Ala Phe Gly Ser Thr Ser
99 AGA GGC AAA AAA GTA TGG ATC AGT TTG CTG TTT GCT TTA GCG TTA ATC TTT ACG ATG GCG TTC GGC AGC ACA TCC

       -80                              -70        PRO                           -60
    Ser Ala Gln Ala Ala Gly Lys Ser Asn Gly Glu Lys Lys Tyr Ile Val Gly Phe Lys Gln Thr Met Ser Thr Met
174 TCT GCC CAG GCG GCA GGG AAA TCA AAC GGG GAA AAG AAA TAT ATT GTC GGG TTT AAA CAG ACA ATG AGC ACG ATG

                            -50                                     -40
    Ser Ala Ala Lys Lys Lys Asp Val Ile Ser Glu Lys Gly Gly Lys Val Gln Lys Gln Phe Lys Tyr Val Asp Ala
249 AGC GCC GCT AAG AAG AAA GAT GTC ATT TCT GAA AAA GGC GGG AAA GTG CAA AAG CAA TTC AAA TAT GTA GAC GCA

        -30                                 -20                                  -10
    Ala Ser Ala Thr Leu Asn Glu Lys Ala Val Lys Glu Leu Lys Lys Asp Pro Ser Val Ala Tyr Val Glu Glu Asp
324 GCT TCA GCT ACA TTA AAC GAA AAA GCT GTA AAA GAA TTG AAA AAA GAC CCG AGC GTC GCT TAC GTT GAA GAA GAT
```

```
                          ┌──► MAT
                        -1  1                                   10
    His Val Ala His Ala Tyr Ala Gln Ser Val Pro Tyr Gly Val Ser Gln Ile Lys Ala Pro Ala Leu His Ser Gln
399 CAC GTA GCA CAT GCG TAC GCG CAG TCC GTG CCT TAC GGC GTA TCA CAA ATT AAA GCC CCT GCT CTG CAC TCT CAA

    20                                      30                                      40
    Gly Tyr Thr Gly Ser Asn Val Lys Val Ala Val Ile Asp Ser Gly Ile Asp Ser Ser His Pro Asp Leu Lys Val
474 GGC TAC ACT GGA TCA AAT GTT AAA GTA GCG GTT ATC GAC AGC GGT ATC GAT TCT TCT CAT CCT GAT TTA AAG GTA

                        50                      Pro Asn         60  Asp
    Ala Gly Gly Ala Ser Met Val Pro Ser Glu Thr Asn Pro Phe Gln Asp Asn Asn Ser His Gly Thr His Val Ala
549 GCA GGC GGA GCC AGC ATG GTT CCT TCT GAA ACA AAT CCT TTC CAA GAC AAC AAC TCT CAC GGA ACT CAC GTT GCC

    70                                      80                              Ser Ala 90
    Gly Thr Val Ala Ala Leu Asn Asn Ser Ile Gly Val Leu Gly Val Ala Pro Ser Ala Ser Leu Tyr Ala Val Lys
624 GGC ACA GTT GCG GCT CTT AAT AAC TCA ATC GGT GTA TTA GGC GTT GCG CCA AGC GCA TCA CTT TAC GCT GTA AAA

                Asp Ala 100                                     110
    Val Leu Gly Ala Asp Gly Ser Gly Gln Tyr Ser Trp Ile Ile Asn Gly Ile Glu Trp Ala Ile Ala Asn Asn Met
699 GTT CTC GGT GCT GAC GGT TCC GGC CAA TAC AGC TGG ATC ATT AAC GGA ATC GAG TGG GCG ATC GCA AAC AAT ATG

    120                                     130                                     140
    Asp Val Ile Asn Met Ser Leu Gly Gly Pro Ser Gly Ser Ala Ala Leu Lys Ala Ala Val Asp Lys Ala Val Ala
774 GAC GTT ATT AAC ATG AGC CTC GGC GGA CCT TCT GGT TCT GCT GCT TTA AAA GCG GCA GTT GAT AAA GCC GTT GCA

                        150                             Ser Thr 160
    Ser Gly Val Val Val Val Ala Ala Ala Gly Asn Glu Gly Thr Ser Gly Ser Ser Ser Thr Val Gly Tyr Pro Gly
849 TCC GGC GTC GTA GTC GTT GCG GCA GCC GGT AAC GAA GGC ACT TCC GGC AGC TCA AGC ACA GTG GGC TAC CCT GGT
```

```
    170                                    180                                    190
    Lys Tyr Pro Ser Val Ile Ala Val Gly Ala Val Asp Ser Ser Asn Gln Arg Ala Ser Phe Ser Ser Val Gly Pro
924 AAA TAC CCT TCT GTC ATT GCA GTA GGC GCT GTT GAC AGC AGC AAC CAA AGA GCA TCT TTC TCA AGC GTA GGA CCT

                        200                                    210
    Glu Leu Asp Val Met Ala Pro Gly Val Ser Ile Gln Ser Thr Leu Pro Gly Asn Lys Tyr Gly Ala Tyr Asn Gly
999 GAG CTT GAT GTC ATG GCA CCT GGC GTA TCT ATC CAA AGC ACG CTT CCT GGA AAC AAA TAC GGG GCG TAC AAC GGT

     220                                    230                                    240
     Thr Ser Met Ala Ser Pro His Val Ala Gly Ala Ala Ala Leu Ile Leu Ser Lys His Pro Asn Trp Thr Asn Thr
1074 ACG TCA ATG GCA TCT CCG CAC GTT GCC GGA GCG GCT GCT TTG ATT CTT TCT AAG CAC CCG AAC TGG ACA AAC ACT

                         250 Gln                           260
     Gln Val Arg Ser Ser Leu Glu Asn Thr Thr Thr Lys Leu Gly Asp Ser Phe Tyr Tyr Gly Lys Gly Leu Ile Asn
1149 CAA GTC CGC AGC AGT TTA GAA AAC ACC ACT ACA AAA CTT GGT GAT TCT TTC TAC TAT GGA AAA GGG CTG ATC AAC

     270                 275                  TERM
     Val Gln Ala Ala Ala Gln OC
1224 GTA CAG GCG GCA GCT CAG TAA  AACATAAAAAACCGGCCTTGGCCCCGCCGGTTTTTTATTATTTTTCTTCCTCCGCATGTTCAATCCGCTCC

1316 ATAATCGACGGATGGCTCCCTCTGAAAATTTTAACGAGAAACGGCGGGTTGACCCGGCTCAGTCCCGTAACGGCCAAGTCCTGAAACGTCTCAATCGCCG

1416 CTTCCCGGTTTCCGGTCAGCTCAATGCCGTAACGGTCGGCGGCGTTTTCCTGATACCGGGAGACGGCATTCGTAATCGGATC
```

*FIG. 1b. (bottom)*

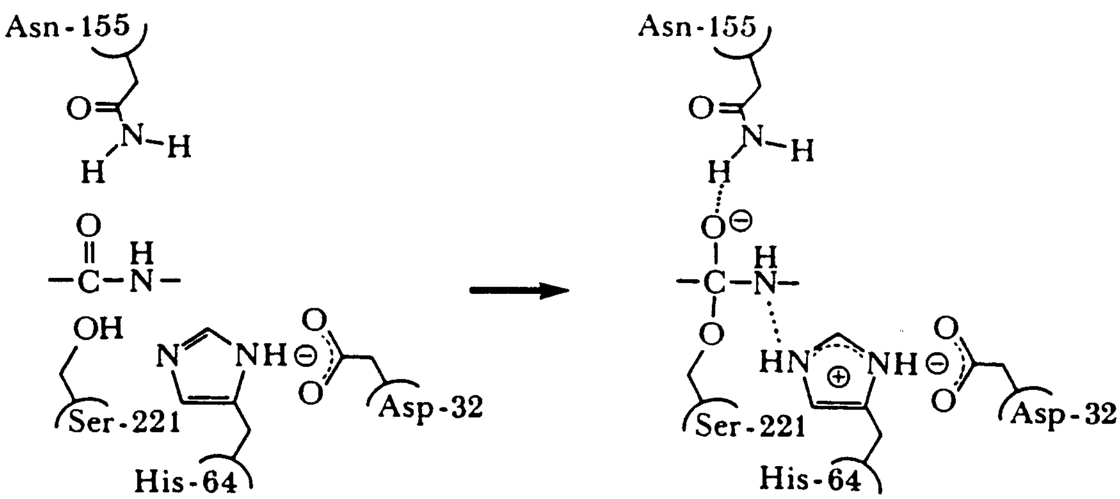

E·S E·S‡

FIG. 2.

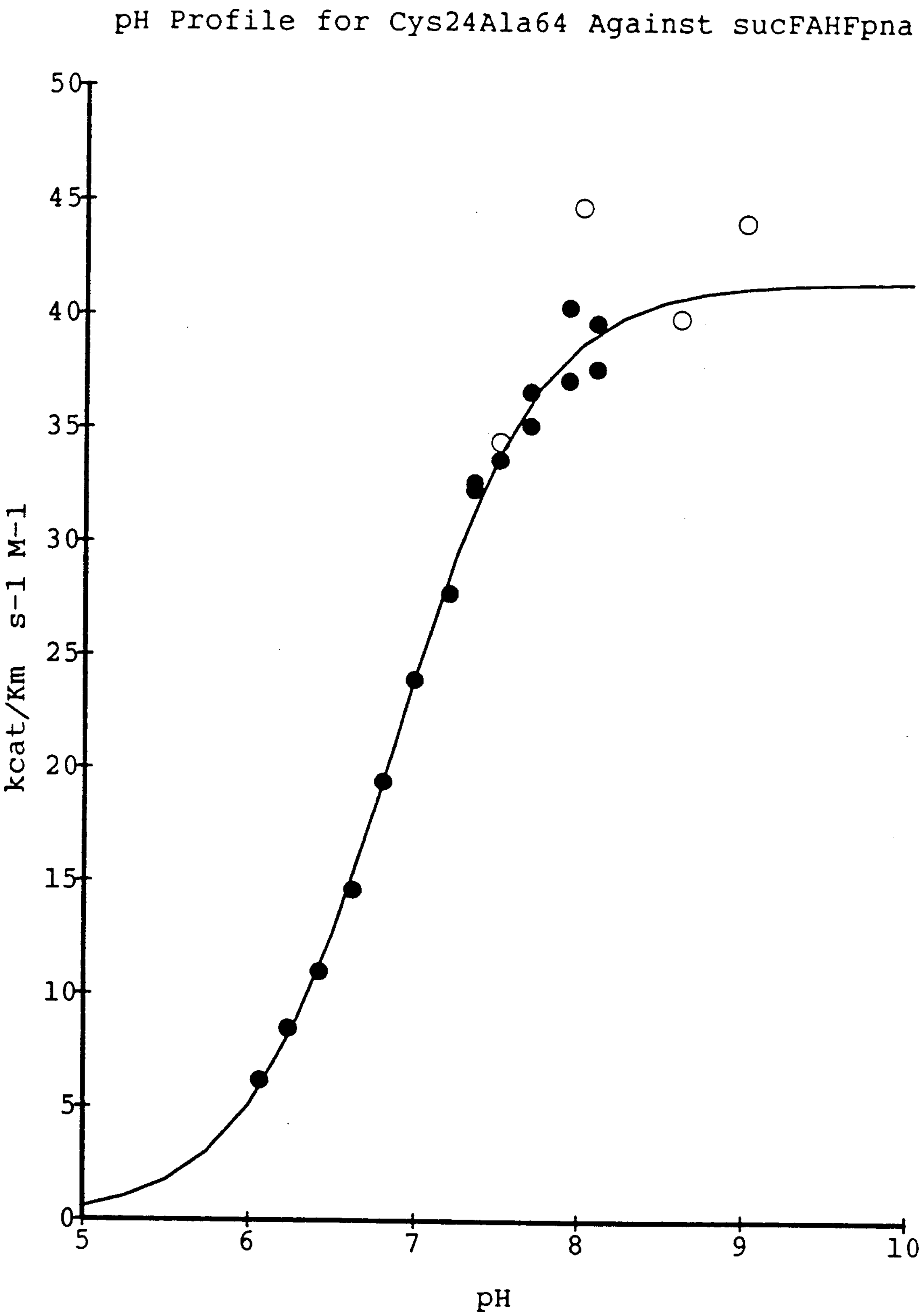
pH Profile for Cys24Ala64 Against sucFAHFpna
kcat/Km s-1 M-1
0
5
10
15
20
25
30
35
40
45
50
5
6
7
8
9
10
pH

FIG. 2a.

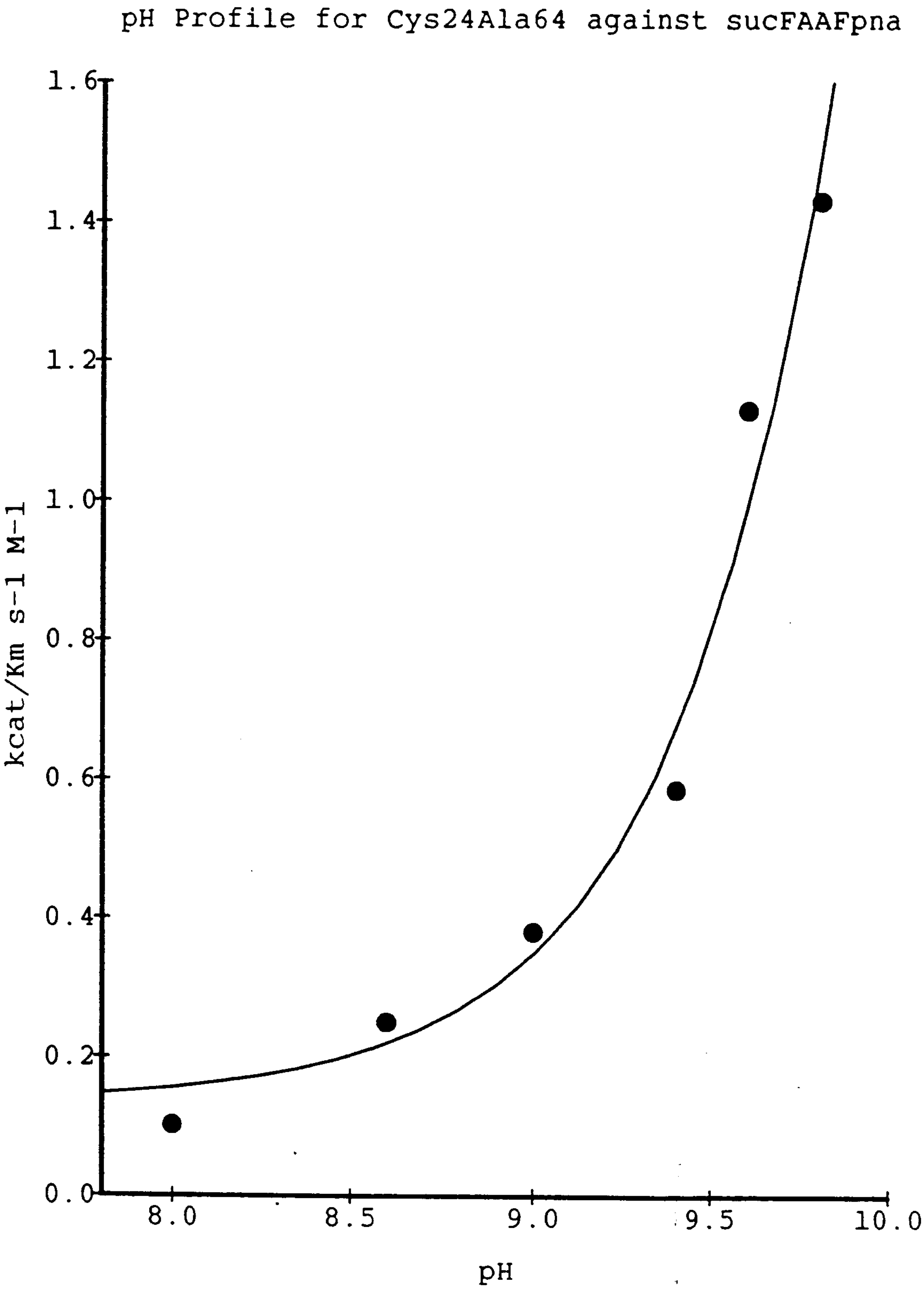
pH Profile for Cys24Ala64 against sucFAAFpna
kcat/Km s-1 M-1
1.6
1.4
1.2
1.0
0.8
0.6
0.4
0.2
0.0
8.0
8.5
9.0
9.5
10.0
pH

FIG. 2b.

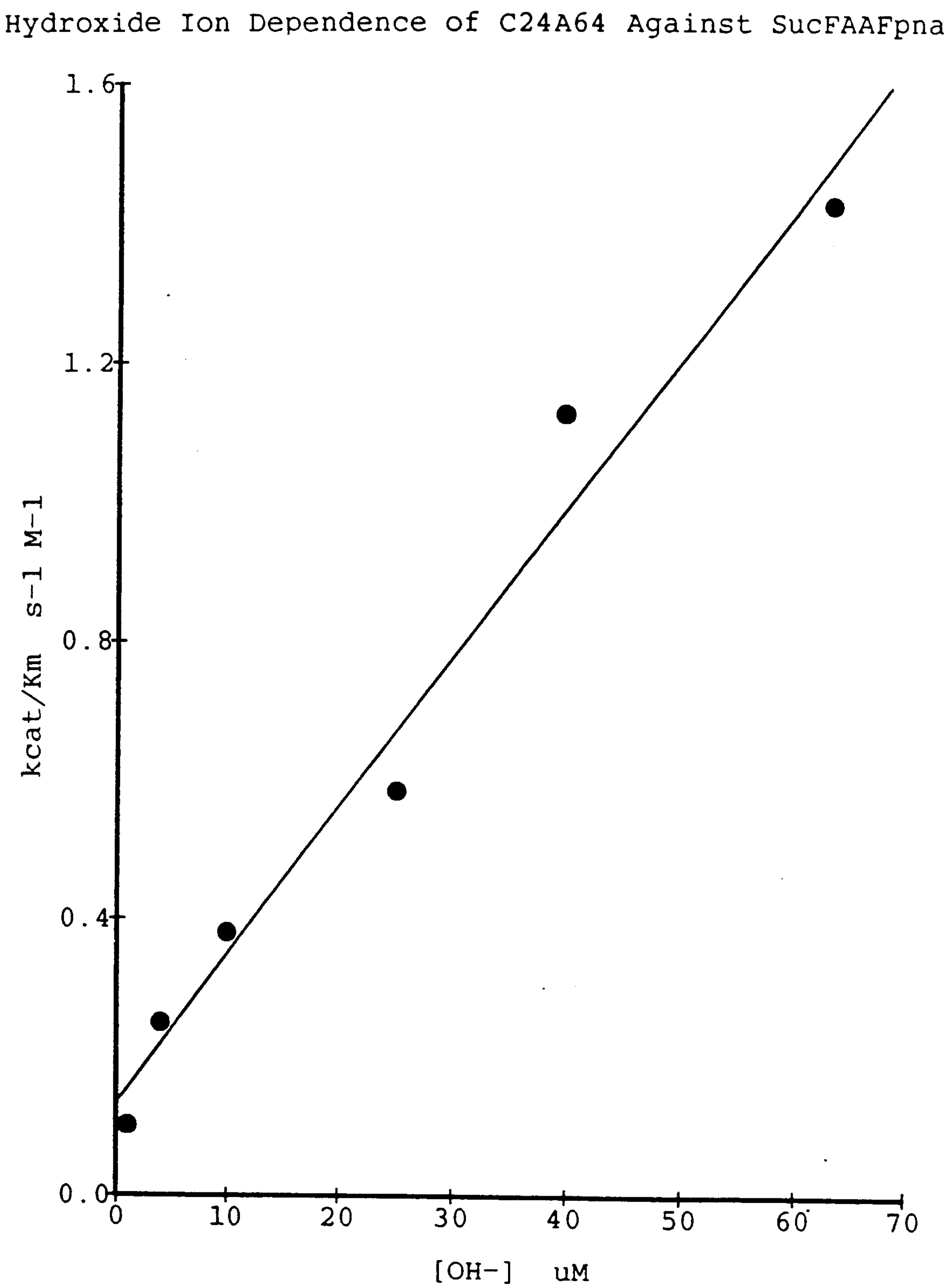
Hydroxide Ion Dependence of C24A64 Against SucFAAFpna
1.6
1.2
0.8
0.4
0.0
kcat/Km s-1 M-1
0
10
20
30
40
50
60
70
[OH-] uM

FIG. 2c.

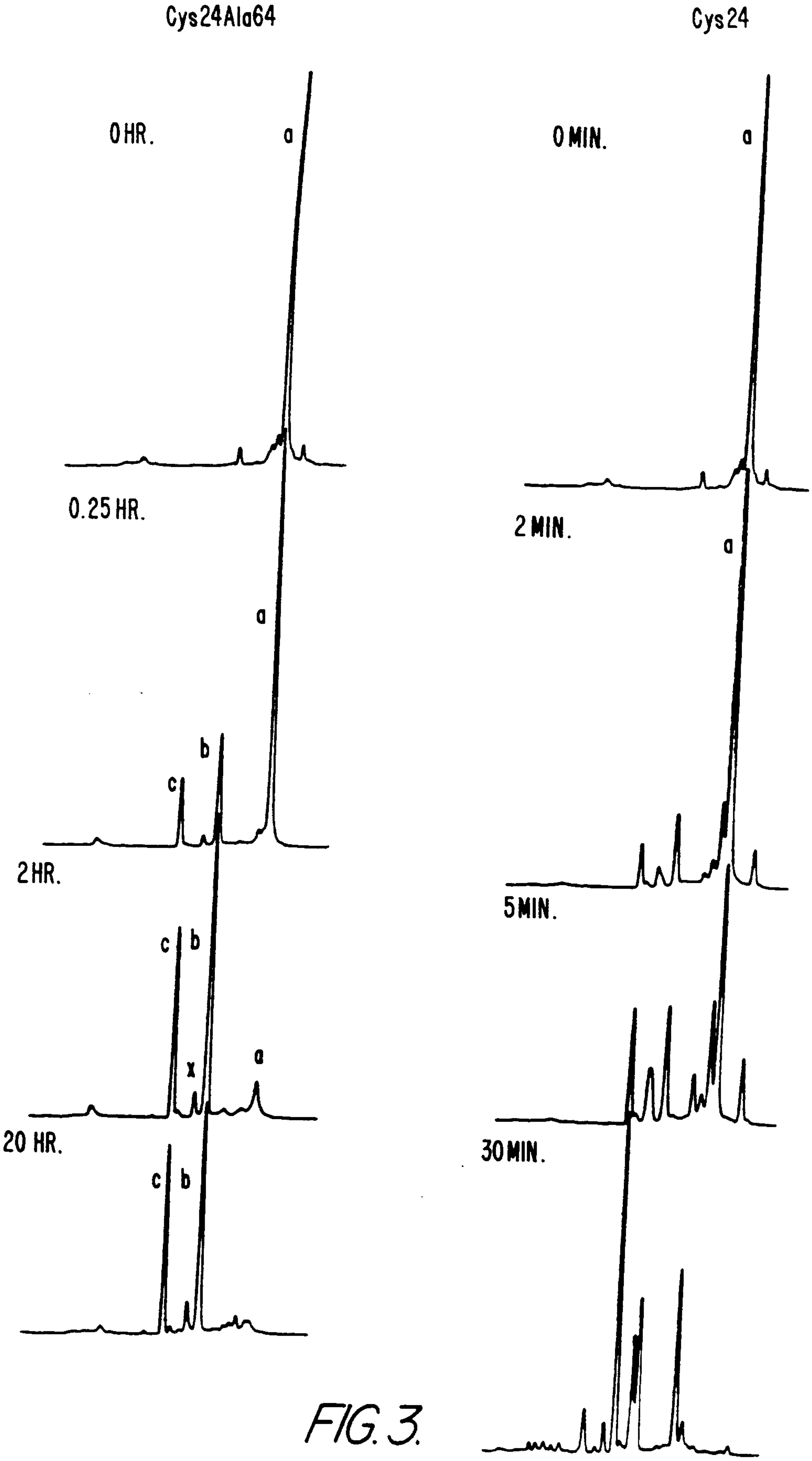
Cys24Ala64
Cys24
0 HR.
a
0 MIN.
a
0.25 HR.
2 MIN.
a
a
b
c
2 HR.
5 MIN.
c
b
x
a
20 HR.
30 MIN.
c
b

FIG. 3.

# FIG. 3a.

Homology of Bacillus proteases
1.Bacillus amyloliquifaciens
2.Bacillus subtilis var.I168
3.Bacillus licheniformis (carlsbergensis)

| | | | | | | | | | | | | | | | | | | | |
|---|---|---|---|---|---|---|---|---|---|---|---|---|---|---|---|---|---|---|---|
| 1 | | | | | | | | | 10 | | | | | | | | | | 20 |
| A | Q | S | V | P | Y | G | V | S | Q | I | K | A | P | A | L | H | S | Q | G |
| A | Q | S | V | P | Y | G | I | S | Q | I | K | A | P | A | L | H | S | Q | G |
| A | Q | T | V | P | Y | G | I | P | L | I | K | A | D | K | V | Q | A | Q | G |
| 21 | | | | | | | | | 30 | | | | | | | | | | 40 |
| Y | T | G | S | N | V | K | V | A | V | I | D | S | G | I | D | S | S | H | P |
| Y | T | G | S | N | V | K | V | A | V | I | D | S | G | I | D | S | S | H | P |
| F | K | G | A | N | V | K | V | A | V | L | D | T | G | I | Q | A | S | H | P |
| 41 | | | | | | | | | 50 | | | | | | | | | | 60 |
| D | L | K | V | A | G | G | A | S | M | V | P | S | E | T | N | P | F | Q | D |
| D | L | N | V | R | G | G | A | S | F | V | P | S | E | T | N | P | Y | Q | D |
| D | L | N | V | V | G | G | A | S | F | V | A | G | E | A | Y | N | T | * | D |

```
61                                     70                                         80
N  N  S  H  G  T  H  V  A  G  T  V  A  A  L  N  N  S  I  G
G  S  S  H  G  T  H  V  A  G  T  I  A  A  L  N  N  S  I  G
G  N  G  H  G  T  H  V  A  G  T  V  A  A  L  D  N  T  T  G

81                                     90                                         100
V  L  G  V  A  P  S  A  S  L  Y  A  V  K  V  L  G  A  D  G
V  L  G  V  S  P  S  A  S  L  Y  A  V  K  V  L  D  S  T  G
V  L  G  V  A  P  S  V  S  L  Y  A  V  K  V  L  N  S  S  G

101                                    110                                        120
S  G  Q  Y  S  W  I  I  N  G  I  E  W  A  I  A  N  N  M  D
S  G  Q  Y  S  W  I  I  N  G  I  E  W  A  I  S  N  N  M  D
S  G  S  Y  S  G  I  V  S  G  I  E  W  A  T  T  N  G  M  D
.PA

121                                    130                                        140
V  I  N  M  S  L  G  G  P  S  G  S  A  A  L  K  A  A  V  D
V  I  N  M  S  L  G  G  P  T  G  S  T  A  L  K  T  V  V  D
V  I  N  M  S  L  G  G  A  S  G  S  T  A  M  K  Q  A  V  D
```

FIG. 3a. (cont.)

141 150 160
K A V A S G V V V V A A A G N E G T S G
K A V S S G I V V A A A A G N E G S S G
N A Y A R G V V V V A A A G N S G N S G

161 170 180
S S S T V G Y P G K Y P S V I A V G A V
S T S T V G Y P A K Y P S T I A V G A V
S T N T I G Y P A K Y D S V I A V G A V

181 190 200
D S S N Q R A S F S S V G P E L D V M A
N S S N Q R A S F S S A G S E L D V M A
D S N S N R A S F S S V G A E L E V M A

201 210 220
P G V S I Q S T L P G N K Y G A Y N G T
P G V S I Q S T L P G G T Y G A Y N G T
P G A G V Y S T Y P T N T Y A T L N G T

FIG. 3a. (cont.)

```
221                                    230                                     240
S  M  A  S  P  H  V  A  G  A  A  A  L  I  L  S  K  H  P  N
S  M  A  T  P  H  V  A  G  A  A  A  L  I  L  S  K  H  P  T
S  M  A  S  P  H  V  A  G  A  A  A  L  I  L  S  K  H  P  N

241                                    250                                     260
W  T  N  T  Q  V  R  S  S  L  E  N  T  T  T  K  L  G  D  S
W  T  N  A  Q  V  R  D  R  L  E  S  T  A  T  Y  L  G  N  S
L  S  A  S  Q  V  R  N  R  L  S  S  T  A  T  Y  L  G  S  S

261                                    270
F  Y  Y  G  K  G  L  I  N  V  Q  A  A  A  Q
F  Y  Y  G  K  G  L  I  N  V  Q  A  A  A  Q
F  Y  Y  G  K  G  L  I  N  V  E  A  A  A  Q
```

FIG. 3a. (cont.)

ALIGNMENT OF B.AMYLOLIQUIFACIENS SUBTILISIN AND THERMITASE
1.B.amyloliquifaciens subtilisin
2.thermitase

FIG. 3b.

```
1                                                               10
A   Q   S   V   *   P   Y   *   *   *   *   *   *   G   V   S   Q   I   K   A
Y   T   P   N   D   P   Y   F   S   S   R   Q   Y   G   P   Q   K   I   Q   A

                        20                                      30
P   A   L   H   S   Q   G   Y   T   G   S   N   V   K   V   A   V   I   D   S
P   Q   A   W   D   I   A   E   *   G   S   G   A   K   I   A   I   V   D   T

                        40                                              50
G   I   D   S   S   H   P   D   L   *   *   K   V   A   G   G   A   S   M   V
G   V   Q   S   N   H   P   D   L   A   G   K   V   V   G   G   W   D   F   V

                                60                                      70
P   S   E   T   N   P   F   Q   D   N   N   S   H   G   T   H   V   A   G   T
D   N   D   S   T   P   *   Q   N   G   N   G   H   G   T   H   C   A   G   I

                                    80                                      90
V   A   A   L   *   N   N   S   I   G   V   L   G   V   A   P   S   A   S   L
A   A   A   V   T   N   N   S   T   G   I   A   G   T   A   P   K   A   S   I
```

```
                                    100                                 110
Y  A  V  K  V  L  G  A  D  G  S  G  Q  Y  S  W  I  I  N  G
L  A  V  R  V  L  D  N  S  G  S  G  T  W  T  A  V  A  N  G

                                    120                                 130
I  E  W  A  I  A  N  N  M  D  V  I  N  M  S  L  G  G  P  S
I  T  Y  A  A  D  Q  G  A  K  V  I  S  L  S  L  G  G  T  V

                                    140                                 150
G  S  A  A  L  K  A  A  V  D  K  A  V  A  S  G  V  V  V  V
G  N  S  G  L  Q  Q  A  V  N  Y  A  W  N  K  G  S  V  V  V
.PA

                                    160                                 170
A  A  A  G  N  E  G  T  S  G  S  S  S  T  V  G  Y  P  G  K
A  A  A  G  N  A  G  N  T  A  *  *  *  *  P  N  Y  P  A  Y

                                    180                                 190
Y  P  S  V  I  A  V  G  A  V  D  S  S  N  Q  R  A  S  F  S
Y  S  N  A  I  A  V  A  S  T  D  Q  N  D  N  K  S  S  F  S
```

FIG. 3b. (cont.)

```
                                    200                                     210
S   V   G   P   E   L   D   V   M   A   P   G   V   S   I   Q   S   T   L   P
T   Y   G   S   V   V   D   V   A   A   P   G   S   W   I   Y   S   T   Y   P

                                    220                                     230
G   N   K   Y   G   A   Y   N   G   T   S   M   A   S   P   H   V   A   G   A
T   S   T   Y   A   S   L   S   G   T   S   M   A   T   P   H   V   A   G   V

                                    240                                     250
A   A   L   I   L   S   K   H   P   N   W   T   N   T   Q   V   R   S   S   L
A   G   L   L   A   S   Q   G   R   S   *   *   A   S   N   I   R   A   A   I

                                        260
E   N   T   T   T   K   *   L   G   D   S   F   Y   Y   G   K   G   L   I   N
E   N   T   A   D   K   I   S   G   T   G   T   Y   W   A   K   G   R   V   N

270
V   Q   A   A   A   Q
A   Y   K   A   V   Q   Y
```

FIG. 3b. (cont.)

FIG. 3c.

TOTALLY CONSERVED RESIDUES IN SUBTILISINS

```
1                                     10                             20
.   .   .   .   P   .   .   .   .   .   .   .   .   .   .   .   .   .   .   .

21                                    30                             40
.   .   G   .   .   .   .   .   .   .   .   D   .   G   .   .   .   .   H   .

41                                    50                             60
.   .   .   .   .   G   .   .   .   .   V   .   .   .   .   .   .   .   .   .

61                                    70                             80
.   .   .   H   G   T   H   .   .   G   .   .   .   .   .   .   .   .   .   .

81                                    90                             100
.   .   G   .   .   .   .   .   .   .   .   .   .   .   V   L   .   .   .   G

101                                   110                            120
S   G   .   .   .   .   .   .   .   G   .   .   .   .   .   .   .   .   .   .

121                                   130                            140
.   .   .   .   .   L   G   .   .   .   .   .   .   .   .   .   .   .   .   .
```

```
141                                     150                                     160
.   .   .   .   .   G   .   .   .   .   .   .   .   G   N   .   .   .   .   .

161                                     170                                     180
.   .   .   .   .   .   Y   P   .   .   .   .   .   .   .   .   V   .   .   .

181                                     190                                     200
.   .   .   .   .   .   .   S   F   S   .   .   .   .   .   .   .   .   .   .

201                                     210                                     220
P   G   .   .   .   .   .   .   .   .   .   .   .   .   .   .   .   .   G   T

221                                     230                                     240
S   M   A   .   P   H   V   A   G   .   .   .   .   .   .   .   .   .   .   .

241                                     250                                     260
.   .   .   .   .   .   R   .   .   .   .   .   .   .   .   .   .   .   .   .

261                                     270
.   .   .   .   .   .   .   .   N   .   .   .   .   .   .
```

FIG. 3c.(cont.)

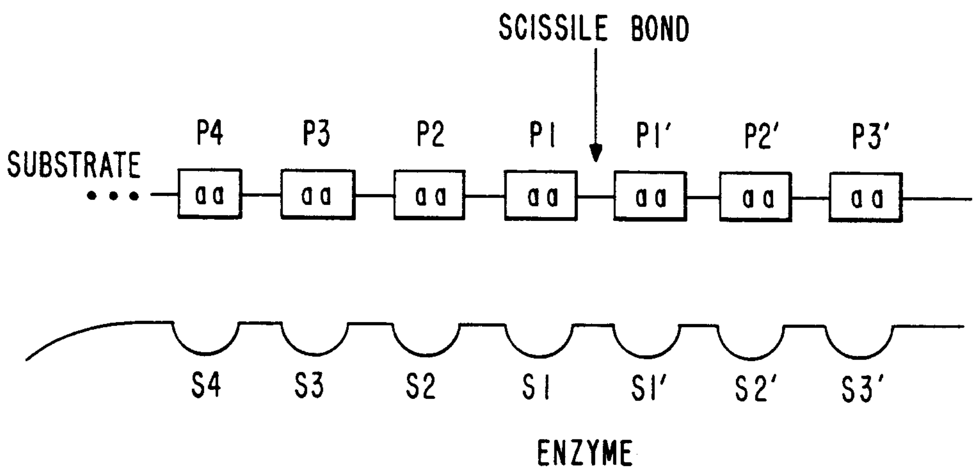
SCISSILE BOND
SUBSTRATE
P4
P3
P2
P1
P1'
P2'
P3'
aa
S4
S3
S2
S1
S1'
S2'
S3'
ENZYME

FIG. 4.

FIG. 5.

B. AMYLOLIQUEFACIENS SUBTILISN ————

PEPTIDE SUBSTRATE —·—·—

CATALITIC TRIAD ($Asp^{32}$, $His^{64}$, $Ser^{221}$) — — —

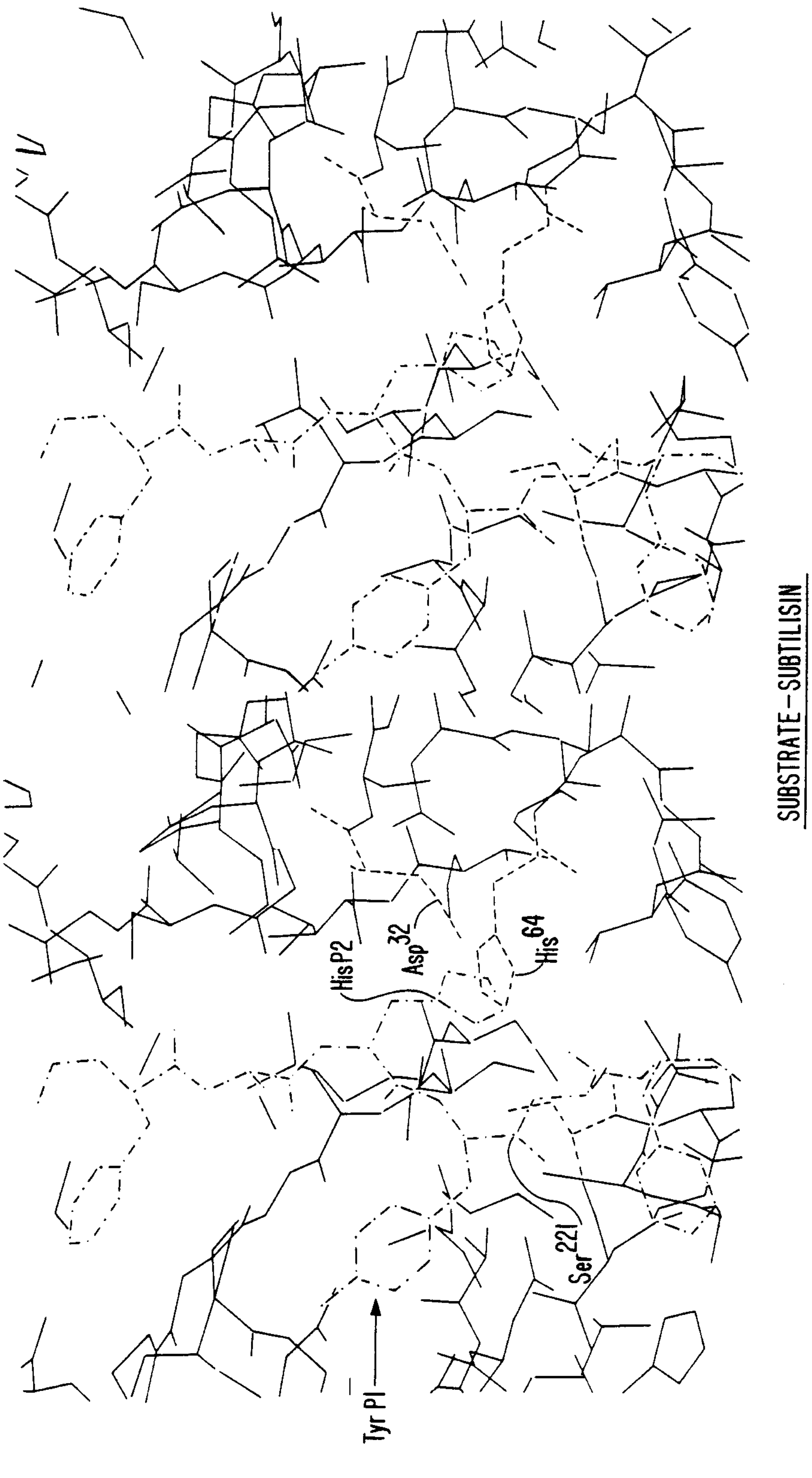

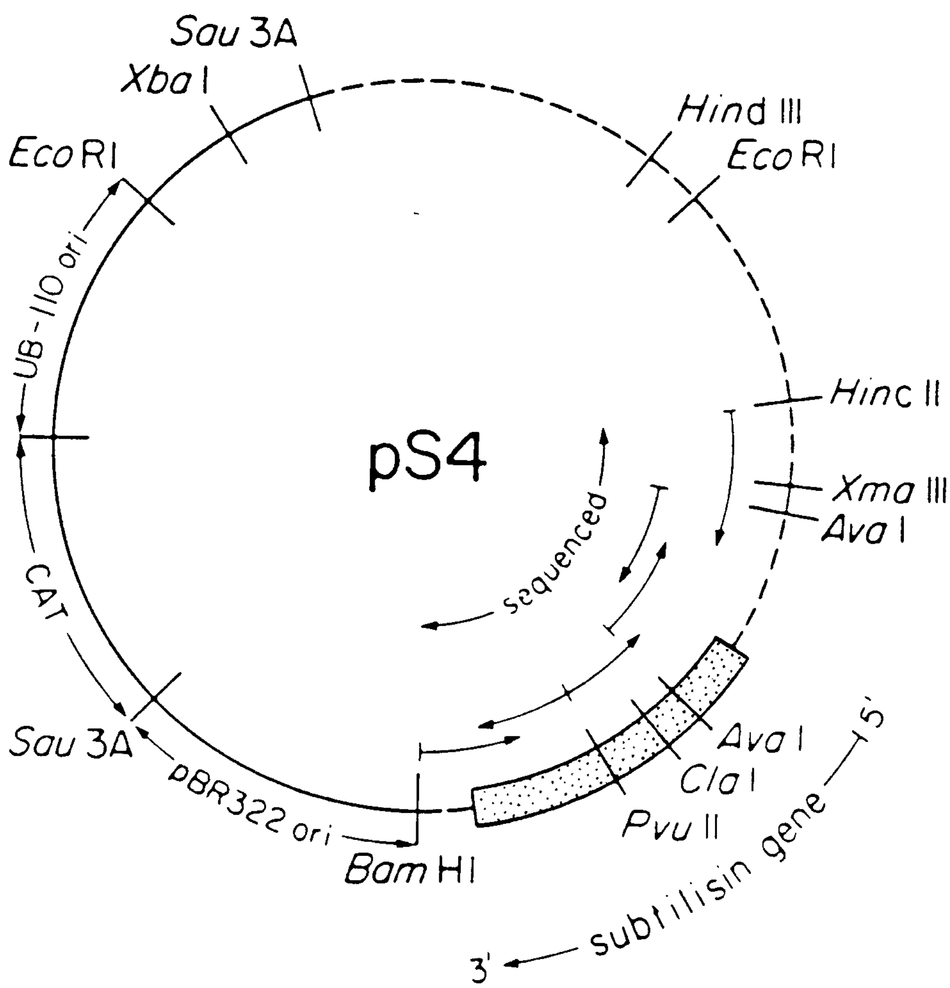
Sau 3A
Xba I
Eco RI
Hind III
Eco RI
UB-110 ori
Hinc II
pS4
Xma III
Ava I
sequenced
CAT
Ava I
Cla I
Sau 3A
pBR322 ori
Pvu II
Bam HI
5'
subtilisin gene
3'

FIG. 6.

FIG.7a.

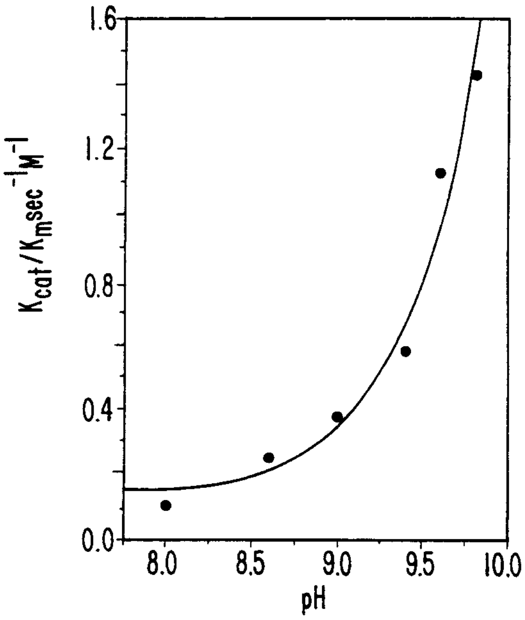


FIG.7c.

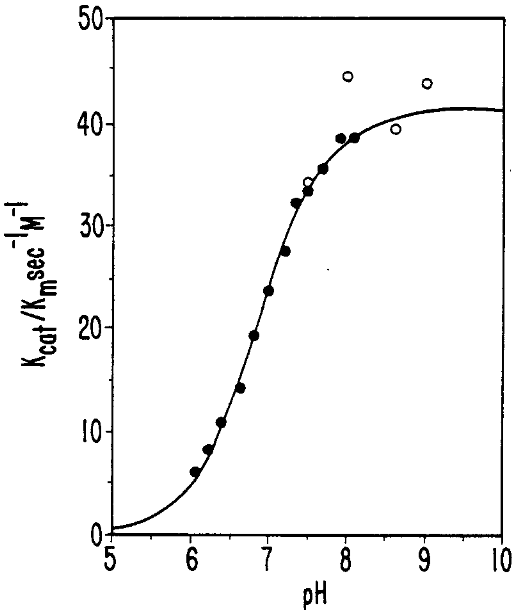


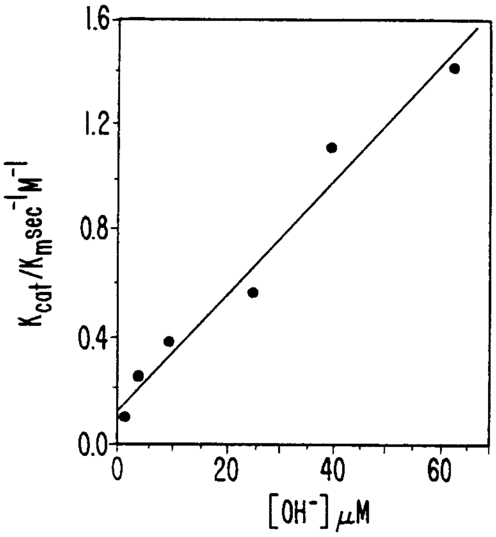


FIG.7b.

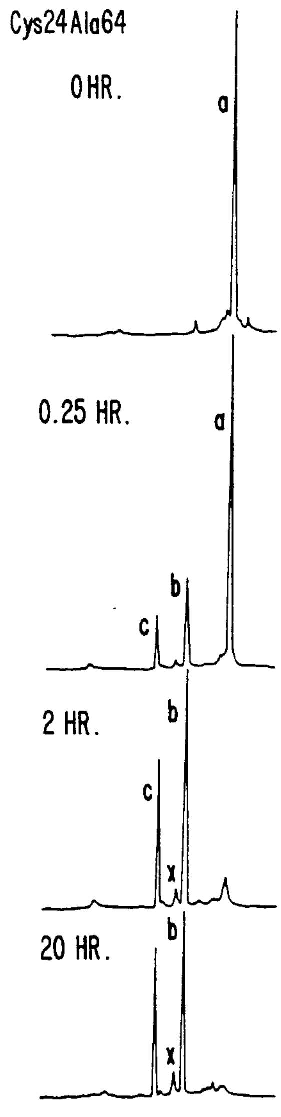
Cys24Ala64
0 HR.
a
0.25 HR.
a
b
c
2 HR.
b
c
x
20 HR.
b
x

FIG. 8a.

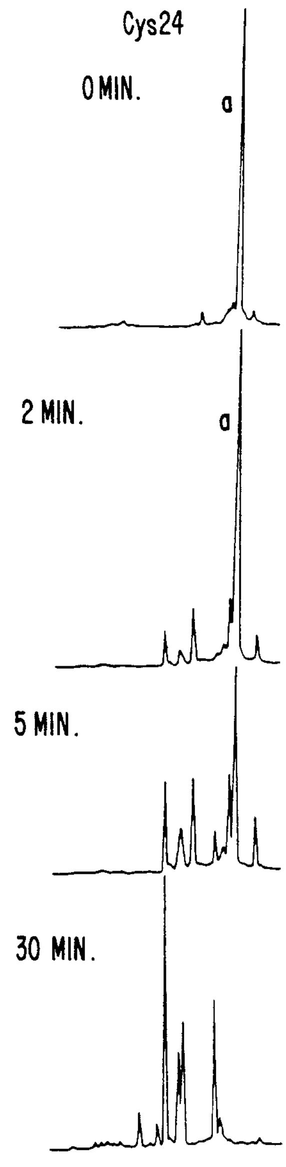
Cys24
0 MIN.
a
2 MIN.
a
5 MIN.
30 MIN.

FIG. 8b.

BOVINE TRYPSIN ————

BOVINE PANCREATIC TRYPSIN INHIBITOR (BPTI) — — — — —

CATALYTIC TRIAD OF TRYPSIN ($Ser^{195}$, $His^{57}$, $Asp^{102}$) —·—·—·—·

PTI-TRYPSIN

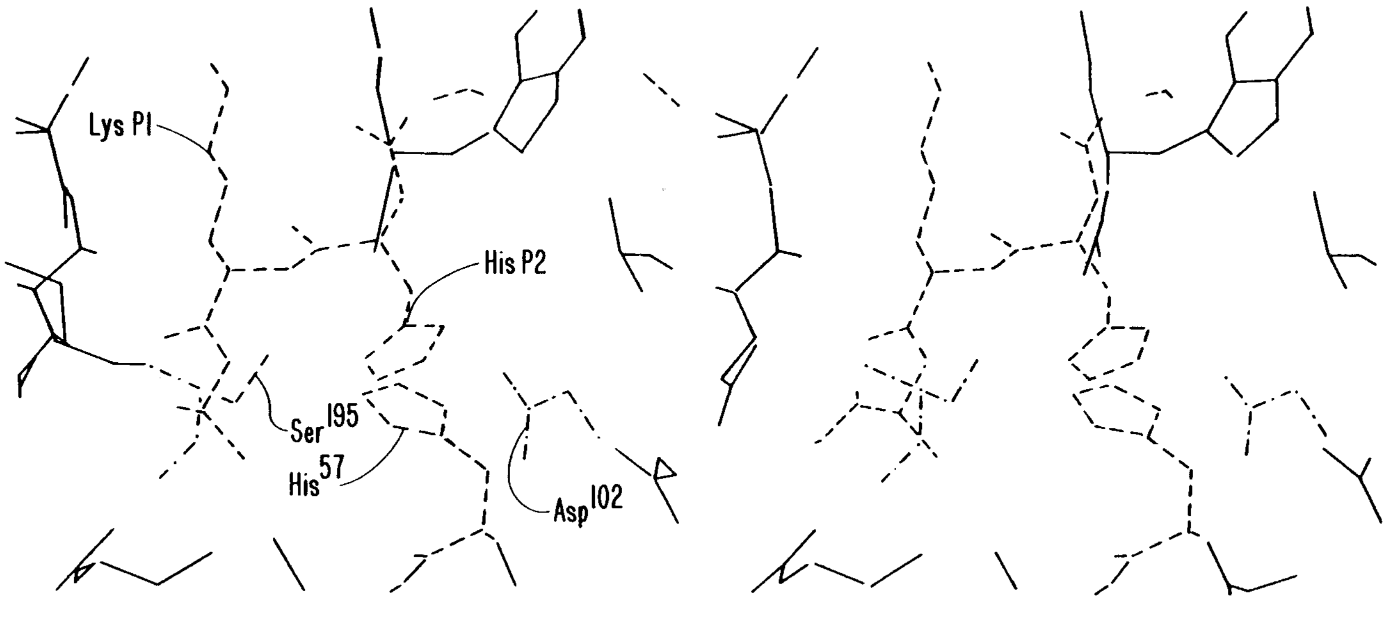


FIG. 9.